(19) Europäisches Patentamt

European Patent Office

Office européen des brevets

(11) **EP 3 800 249 B1**

(12) **EUROPEAN PATENT SPECIFICATION**

(45) Date of publication and mention
of the grant of the patent:
**22.06.2022   Bulletin 2022/25**

(21) Application number: **20198764.1**

(22) Date of filing: **08.02.2017**

(51) International Patent Classification (IPC):
*C12N 9/22* *(2006.01)*    *C12P 19/34* *(2006.01)*
*C40B 30/04* *(2006.01)*    *C40B 40/06* *(2006.01)*
*C12Q 1/68* *(2018.01)*    *C12Q 1/6834* *(2018.01)*
*C12Q 1/6832* *(2018.01)*    *C12Q 1/6818* *(2018.01)*

(52) Cooperative Patent Classification (CPC):
(C-Sets available)
**C12N 9/22; C12Q 1/6818; C12Q 1/6832;
C12Q 1/6834**                    (Cont.)

(54) **DETECTION OF NUCLEIC ACIDS**

NACHWEIS VON NUKLEINSÄUREN

DÉTECTION D'ACIDES NUCLÉIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.02.2016   US 201662293589 P**

(43) Date of publication of application:
**07.04.2021   Bulletin 2021/14**

(62) Document number(s) of the earlier application(s) in
accordance with Art. 76 EPC:
**17750678.9 / 3 414 327**

(73) Proprietor: **The Regents of The University of
Michigan
Ann Arbor, Michigan 48109-2590 (US)**

(72) Inventors:
• **WALTER, Nils**
**Ann Arbor, Michigan 48109-2590 (US)**
• **TEWARI, Muneesh**
**Ann Arbor, Michigan 48109-2590 (US)**
• **JOHNSON-BUCK, Alexander**
**Ann Arbor, Michigan 48109-2590 (US)**

(74) Representative: **Glawe, Delfs, Moll
Partnerschaft mbB von
Patent- und Rechtsanwälten
Postfach 13 03 91
20103 Hamburg (DE)**

(56) References cited:
**WO-A1-2016/025477**

• **JOHNSON-BUCK ALEXANDER ET AL: "Kinetic
fingerprinting to identify and count single nucleic
acids", NATURE BIOTECHNOLOGY, vol. 33, no.
7, 22 June 2015 (2015-06-22), pages 730-732,
XP055776096, us ISSN: 1087-0156, DOI:
10.1038/nbt.3246 Retrieved from the Internet:
URL:http://www.nature.com/articles/nbt.324 6>**

**EP 3 800 249 B1**

**(Cont. next page)**

(52) Cooperative Patent Classification (CPC): (Cont.)

C-Sets
**C12Q 1/6818, C12Q 2527/107, C12Q 2537/162,**
**C12Q 2543/10, C12Q 2565/101, C12Q 2565/518;**
**C12Q 1/6832, C12Q 2521/301, C12Q 2525/113,**
**C12Q 2525/207, C12Q 2565/107;**
**C12Q 1/6834, C12Q 2521/301, C12Q 2525/113,**
**C12Q 2525/207, C12Q 2565/107**

SEQUENCE LISTING

<110> THE REGENTS OF THE UNIVERSITY OF MICHIGAN

<120> DETECTION OF NUCLEIC ACIDS

<130> UNIG172PEP02

<140> EP
<141> 2017-02-08

<150> EP17750678.9
<151> 2017-02-08

<150> PCT/US2017/016977
<151> 2017-02-08

<150> US 62/293,589
<151> 2016-02-10

<160> 1

<170> PatentIn version 3.5

<210> 1
<211> 1368
<212> PRT
<213> Streptococcus pyogenes

<400> 1

```
Met Asp Lys Lys Tyr Ser Ile Gly Leu Ala Ile Gly Thr Asn Ser Val
1                   5                   10                  15

Gly Trp Ala Val Ile Thr Asp Glu Tyr Lys Val Pro Ser Lys Lys Phe
            20                  25                  30

Lys Val Leu Gly Asn Thr Asp Arg His Ser Ile Lys Lys Asn Leu Ile
            35                  40                  45

Gly Ala Leu Leu Phe Asp Ser Gly Glu Thr Ala Glu Ala Thr Arg Leu
    50                  55                  60

Lys Arg Thr Ala Arg Arg Arg Tyr Thr Arg Arg Lys Asn Arg Ile Cys
65                  70                  75                  80

Tyr Leu Gln Glu Ile Phe Ser Asn Glu Met Ala Lys Val Asp Asp Ser
                85                  90                  95

Phe Phe His Arg Leu Glu Glu Ser Phe Leu Val Glu Glu Asp Lys Lys
            100                 105                 110

His Glu Arg His Pro Ile Phe Gly Asn Ile Val Asp Glu Val Ala Tyr
            115                 120                 125
```

```
His Glu Lys Tyr Pro Thr Ile Tyr His Leu Arg Lys Lys Leu Val Asp
    130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                165                 170                 175

Asp Asn Ser Asp Val Asp Lys Leu Phe Ile Gln Leu Val Gln Thr Tyr
                180                 185                 190

Asn Gln Leu Phe Glu Glu Asn Pro Ile Asn Ala Ser Gly Val Asp Ala
            195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
            275                 280                 285

Leu Phe Leu Ala Ala Lys Asn Leu Ser Asp Ala Ile Leu Leu Ser Asp
    290                 295                 300

Ile Leu Arg Val Asn Thr Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
                340                 345                 350

Asp Gln Ser Lys Asn Gly Tyr Ala Gly Tyr Ile Asp Gly Gly Ala Ser
            355                 360                 365

Gln Glu Glu Phe Tyr Lys Phe Ile Lys Pro Ile Leu Glu Lys Met Asp
    370                 375                 380
```

```
Gly Thr Glu Glu Leu Leu Val Lys Leu Asn Arg Glu Asp Leu Leu Arg
385             390         395             400

Lys Gln Arg Thr Phe Asp Asn Gly Ser Ile Pro His Gln Ile His Leu
            405             410             415

Gly Glu Leu His Ala Ile Leu Arg Arg Gln Glu Asp Phe Tyr Pro Phe
        420             425             430

Leu Lys Asp Asn Arg Glu Lys Ile Glu Lys Ile Leu Thr Phe Arg Ile
        435             440             445

Pro Tyr Tyr Val Gly Pro Leu Ala Arg Gly Asn Ser Arg Phe Ala Trp
    450             455             460

Met Thr Arg Lys Ser Glu Glu Thr Ile Thr Pro Trp Asn Phe Glu Glu
465             470             475             480

Val Val Asp Lys Gly Ala Ser Ala Gln Ser Phe Ile Glu Arg Met Thr
            485             490             495

Asn Phe Asp Lys Asn Leu Pro Asn Glu Lys Val Leu Pro Lys His Ser
            500             505             510

Leu Leu Tyr Glu Tyr Phe Thr Val Tyr Asn Glu Leu Thr Lys Val Lys
        515             520             525

Tyr Val Thr Glu Gly Met Arg Lys Pro Ala Phe Leu Ser Gly Glu Gln
    530             535             540

Lys Lys Ala Ile Val Asp Leu Leu Phe Lys Thr Asn Arg Lys Val Thr
545             550             555             560

Val Lys Gln Leu Lys Glu Asp Tyr Phe Lys Lys Ile Glu Cys Phe Asp
            565             570             575

Ser Val Glu Ile Ser Gly Val Glu Asp Arg Phe Asn Ala Ser Leu Gly
            580             585             590

Thr Tyr His Asp Leu Leu Lys Ile Ile Lys Asp Lys Asp Phe Leu Asp
        595             600             605

Asn Glu Glu Asn Glu Asp Ile Leu Glu Asp Ile Val Leu Thr Leu Thr
        610             615             620

Leu Phe Glu Asp Arg Glu Met Ile Glu Glu Arg Leu Lys Thr Tyr Ala
625             630             635             640
```

34

```
His Leu Phe Asp Asp Lys Val Met Lys Gln Leu Lys Arg Arg Arg Tyr
             645             650             655

Thr Gly Trp Gly Arg Leu Ser Arg Lys Leu Ile Asn Gly Ile Arg Asp
             660             665             670

Lys Gln Ser Gly Lys Thr Ile Leu Asp Phe Leu Lys Ser Asp Gly Phe
             675             680             685

Ala Asn Arg Asn Phe Met Gln Leu Ile His Asp Asp Ser Leu Thr Phe
             690             695             700

Lys Glu Asp Ile Gln Lys Ala Gln Val Ser Gly Gln Gly Asp Ser Leu
705             710             715             720

His Glu His Ile Ala Asn Leu Ala Gly Ser Pro Ala Ile Lys Lys Gly
             725             730             735

Ile Leu Gln Thr Val Lys Val Val Asp Glu Leu Val Lys Val Met Gly
             740             745             750

Arg His Lys Pro Glu Asn Ile Val Ile Glu Met Ala Arg Glu Asn Gln
             755             760             765

Thr Thr Gln Lys Gly Gln Lys Asn Ser Arg Glu Arg Met Lys Arg Ile
     770             775             780

Glu Glu Gly Ile Lys Glu Leu Gly Ser Gln Ile Leu Lys Glu His Pro
785             790             795             800

Val Glu Asn Thr Gln Leu Gln Asn Glu Lys Leu Tyr Leu Tyr Tyr Leu
             805             810             815

Gln Asn Gly Arg Asp Met Tyr Val Asp Gln Glu Leu Asp Ile Asn Arg
             820             825             830

Leu Ser Asp Tyr Asp Val Asp Ala Ile Val Pro Gln Ser Phe Leu Lys
             835             840             845

Asp Asp Ser Ile Asp Asn Lys Val Leu Thr Arg Ser Asp Lys Asn Arg
     850             855             860

Gly Lys Ser Asp Asn Val Pro Ser Glu Glu Val Val Lys Lys Met Lys
865             870             875             880

Asn Tyr Trp Arg Gln Leu Leu Asn Ala Lys Leu Ile Thr Gln Arg Lys
```

                            885                    890                    895


Phe Asp Asn Leu Thr Lys Ala Glu Arg Gly Gly Leu Ser Glu Leu Asp
            900                 905                 910


Lys Ala Gly Phe Ile Lys Arg Gln Leu Val Glu Thr Arg Gln Ile Thr
            915                 920                 925


Lys His Val Ala Gln Ile Leu Asp Ser Arg Met Asn Thr Lys Tyr Asp
    930                 935                 940


Glu Asn Asp Lys Leu Ile Arg Glu Val Lys Val Ile Thr Leu Lys Ser
945                 950                 955                 960


Lys Leu Val Ser Asp Phe Arg Lys Asp Phe Gln Phe Tyr Lys Val Arg
                965                 970                 975


Glu Ile Asn Asn Tyr His His Ala His Asp Ala Tyr Leu Asn Ala Val
            980                 985                 990


Val Gly Thr Ala Leu Ile Lys Lys  Tyr Pro Lys Leu Glu  Ser Glu Phe
        995                 1000                 1005


Val Tyr  Gly Asp Tyr Lys Val  Tyr Asp Val Arg Lys  Met Ile Ala
    1010                 1015                 1020


Lys Ser  Glu Gln Glu Ile Gly  Lys Ala Thr Ala Lys  Tyr Phe Phe
    1025                 1030                 1035


Tyr Ser  Asn Ile Met Asn Phe  Phe Lys Thr Glu Ile  Thr Leu Ala
    1040                 1045                 1050


Asn Gly  Glu Ile Arg Lys Arg  Pro Leu Ile Glu Thr  Asn Gly Glu
    1055                 1060                 1065


Thr Gly  Glu Ile Val Trp Asp  Lys Gly Arg Asp Phe  Ala Thr Val
    1070                 1075                 1080


Arg Lys  Val Leu Ser Met Pro  Gln Val Asn Ile Val  Lys Lys Thr
    1085                 1090                 1095


Glu Val  Gln Thr Gly Gly Phe  Ser Lys Glu Ser Ile  Leu Pro Lys
    1100                 1105                 1110


Arg Asn  Ser Asp Lys Leu Ile  Ala Arg Lys Lys Asp  Trp Asp Pro
    1115                 1120                 1125

```
Lys Lys  Tyr Gly Gly Phe Asp  Ser Pro Thr Val Ala  Tyr Ser Val
    1130              1135              1140

Leu Val  Val Ala Lys Val Glu  Lys Gly Lys Ser Lys  Lys Leu Lys
    1145              1150              1155

Ser Val  Lys Glu Leu Leu Gly  Ile Thr Ile Met Glu  Arg Ser Ser
    1160              1165              1170

Phe Glu  Lys Asn Pro Ile Asp  Phe Leu Glu Ala Lys  Gly Tyr Lys
    1175              1180              1185

Glu Val  Lys Lys Asp Leu Ile  Ile Lys Leu Pro Lys  Tyr Ser Leu
    1190              1195              1200

Phe Glu  Leu Glu Asn Gly Arg  Lys Arg Met Leu Ala  Ser Ala Gly
    1205              1210              1215

Glu Leu  Gln Lys Gly Asn Glu  Leu Ala Leu Pro Ser  Lys Tyr Val
    1220              1225              1230

Asn Phe  Leu Tyr Leu Ala Ser  His Tyr Glu Lys Leu  Lys Gly Ser
    1235              1240              1245

Pro Glu  Asp Asn Glu Gln Lys  Gln Leu Phe Val Glu  Gln His Lys
    1250              1255              1260

His Tyr  Leu Asp Glu Ile Ile  Glu Gln Ile Ser Glu  Phe Ser Lys
    1265              1270              1275

Arg Val  Ile Leu Ala Asp Ala  Asn Leu Asp Lys Val  Leu Ser Ala
    1280              1285              1290

Tyr Asn  Lys His Arg Asp Lys  Pro Ile Arg Glu Gln  Ala Glu Asn
    1295              1300              1305

Ile Ile  His Leu Phe Thr Leu  Thr Asn Leu Gly Ala  Pro Ala Ala
    1310              1315              1320

Phe Lys  Tyr Phe Asp Thr Thr  Ile Asp Arg Lys Arg  Tyr Thr Ser
    1325              1330              1335

Thr Lys  Glu Val Leu Asp Ala  Thr Leu Ile His Gln  Ser Ile Thr
    1340              1345              1350

Gly Leu  Tyr Glu Thr Arg Ile  Asp Leu Ser Gln Leu  Gly Gly Asp
    1355              1360              1365
```

37

**Description**

This application claims priority to United States provisional patent application serial number 62/293,589, filed February 10, 2016.

**FIELD**

Provided herein is technology relating to detecting and identifying nucleic acids and particularly, but not exclusively, to compositions, methods, kits, and systems for detecting, identifying, and quantifying target nucleic acids with high confidence at single-molecule resolution.

**BACKGROUND**

Early detection is critical to the effective treatment of many diseases, especially cancer. Research related to identifying detectable biomarkers associated with early-stage disease has indicated that nucleic acids provide highly specific biomarkers of cancer and other maladies. For example, cancer cell-derived double-stranded DNA (dsDNA) together with secondary structured long non-coding RNA (lncRNA) have recently emerged as sensitive and specific biomarkers of cancer and other diseases in crude human biofluids such as blood, urine, and sputum.

However, despite their promise as diagnostic biomarkers, the sensitive and specific detection of nucleic acid biomarkers has proven challenging. In particular, existing techniques for detecting nucleic acids utilize probes that form a thermodynamically stable complex with the target molecule and are thus limited to weak and often unreliable thermodynamic discrimination against background signal, spurious targets, or closely related mutant nucleic acids. In addition, the presence of a complementary DNA or RNA strand in the sample severely limits accessibility of the target sequence.

Thus, a sensitive and specific assay for the amplification-free detection of nucleic acids in minimally treated native biofluids is needed to provide a rapid and reliable identification and/or quantification of nucleic acid biomarkers.

Kinetic fingerprinting to identify and count single nucleic acids is known from Johnson-Buck et al. (2015) "Kinetic fingerprinting to identify and count single nucleic acids" Nat Biotechnol 33: 730-2. Johnson-Bucket *et al.* discloses a kinetic fingerprinting approach called Single Molecule Recognition through Equilibrium Poisson sampling (SiMREPS) for the amplification-free counting of single unlabeled miRNA molecules, which circumvents thermodynamic limits of specificity and virtually eliminates false positives.

**SUMMARY**

The invention is set out in the appended claims. The embodiments of the description which do not fall within the scope of said claims are provided for illustrative purposes only and do not form part of the present invention. For the sake of clarity, the subject-matter described in the present specification not corresponding to the complex as defined in claims 1-6 and the methods as defined in claims 7-15 of the enclosed claim set are not part of the present invention. Accordingly, provided herein is a technology for the specific and ultrasensitive detection and counting of single nucleic acid (e.g., dsDNA, lncRNA, methylated DNA, etc.) target molecules based on the transient binding of short labeled probes to a target nucleic acid. The target nucleic acid is detected by a kinetic "fingerprint" signal produced by the probe-target interaction as defined in the claims. This Single-Molecule Recognition with Equilibrium Poisson Sampling (SiMREPS) technology provides for the sensitive detection of both single-stranded nucleic acids (see, e.g., US 2016/0046988 A1) and double-stranded nucleic acids. Detection of double-stranded nucleic acids may comprise use of dCas9-guided capture and DNA melting. This technology may comprise the capture of unlabeled targets on a glass or fused silica surface using a guideRNA (gRNA)-loaded, catalytically inactive ("dead") dCas9 enzyme or enzymes that bind one or more segments of the target nucleic acid with high specificity. In some examples (e.g., for detecting lncRNA), the technology further comprises use of a protospacer adjacent motif (PAM) oligonucleotide to provide for dCas9 targeting of nucleic acid targets (e.g., lncRNA).

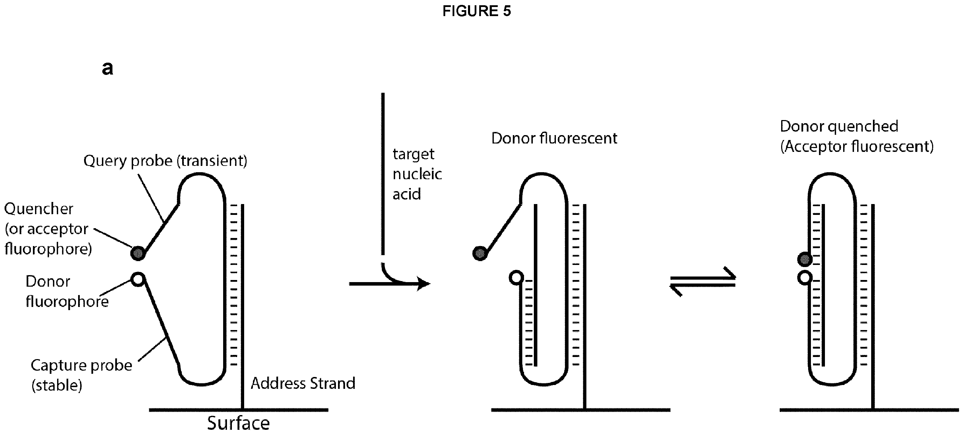

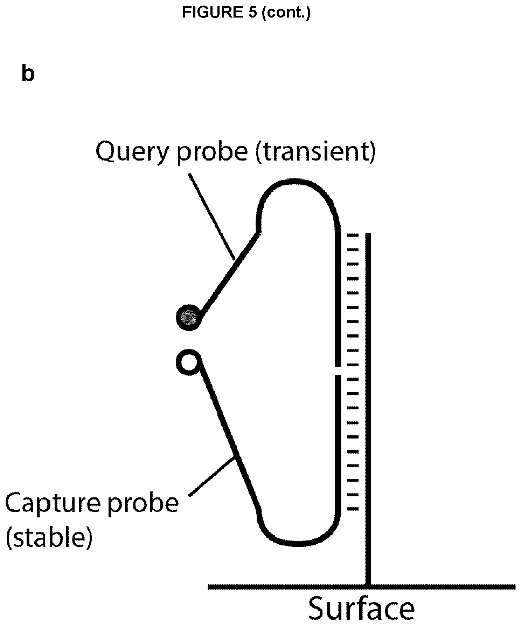

The technology may comprise use of an intramolecular SiMREPS probe, e.g., a capture probe and a query probe that are linked to provide an intramolecular probing mechanism (see, e.g., Fig. 5 a and b; see infra).

More broadly, in some examples, one or more complexes (e.g., one or more dCas9/gRNA complexes) recognize specific segments (e.g., nucleotide sequences) in a target nucleic acid to immobilize the target nucleic acid to a surface. Furthermore, examples provide that these same or other complexes and/or complementary oligonucleotides melt double-stranded regions of the target nucleic acid to provide access for the binding of a labeled query probe (e.g., for binding of the query probe to a second segment, e.g., a query region). Surface capture (and, in some examples, melting of one or more double-stranded regions) is followed by observation of the repeated, transient binding of a short fluorescently labeled DNA query probe to the second segment (e.g., query region) of the target nucleic acid that has been made accessible by dCas9-mediated melting.

Furthermore, in addition to using dCas9/gRNA for immobilization and/or exposure of target sequences (e.g., one or more query regions), examples also provide a technology in which a labeled (e.g., fluorescently labeled) dCas9/gRNA complex provides detection of the target nucleic acid. In particular, the dCas9/gRNA complex provides a query probe for SiMREPS, since the dwell time of dCas9/gRNA on a DNA sequence is sensitive to the number of base pairs formed between the gRNA and the target DNA sequence. Examples of the technology provide that the number of base pairs formed between the gRNA and the target DNA is tuned to promote rapid dissociation from mutant sequences but slow dissociation from wild-type sequences or vice-versa. Furthermore, engineered dCas9 proteins provide the appropriate kinetics and sequence specificity (see, e.g., Kleinstiver et al. (2016) "High-fidelity CRISPR-Cas9 nucleases with no detectable genome-wide off-target effects" Nature 529: 490-495; Slaymaker et al. (2015) "Rationally engineered Cas9 nucleases with improved specificity" Science 351: 84-8) describing engineered Cas9 proteins that interact more weakly with the DNA backbone than native Cas9, thus reducing off-target effects.

In particular examples described herein, the SiMREPS probes repeatedly bind to a target sequence (e.g., query region) specifically made accessible by binding of dCas9/gRNA to one or more nucleic acid region(s) adjacent to the target region (e.g., "adjacent regions"). The repeated binding of the query probes to the query region provides a unique, continuous kinetic "fingerprint", providing a large number of independent measurements for each observed target molecule. This repeated kinetic sampling affords two main advantages: (1) arbitrarily high discrimination against background signals with increased sampling time, essentially eliminating false positive signals; and (2) exquisite sensitivity to subtle differences in the identity of the molecular target, allowing for discrimination of slightly different biomarkers (e.g., differing by only one DNA/RNA base of a disease-related mutation, a methylation pattern or other chemical marks) with very high confidence. In addition, as a single-molecule technique, this approach detects a small amount of target molecule (such as derived from a single cell) in the presence of a large excess of a closely related, but spurious (e.g., mutant) target. In contrast to techniques requiring PCR amplification (the current standard for low-abundance targets), this technique utilizes no target amplification and hence requires pre-treatment of biological samples only with, e.g., dCas9/gRNA at ambient temperature prior to target detection, avoiding the introduction of sampling bias. In addition, as a direct detection technique, it does not lose any chemical marks on the target as amplification-based detection approaches often do. The technique finds use, for example, in the diagnosis of cancer from circulating tumor DNA and lncRNA in human blood serum. The technology finds use also in drug and antibiotic resistance gene detection in pathogens and human tumor cells.

Accordingly, provided herein are examples of a complex for providing a detectable fingerprint of a double-stranded target nucleic acid, the complex comprising a double-stranded target nucleic acid (e.g., a DNA, an RNA, a DNA/RNA hybrid) comprising a first region adjacent to a second region; a melting component (e.g., an immobilized melting component) interacting with the first region to form a thermodynamically stable complex and provide the second region in a single-stranded form; and a query probe that binds repeatedly to the second region to provide a detectable fingerprint associated with the double-stranded target nucleic acid. The double-stranded nucleic acid can be a single-stranded nucleic acid that comprises a region having a double-stranded secondary structure. The melting component may comprise a dCas9. The melting component may comprise a single-stranded binding protein. The melting component may be a protein and in some examples the melting component is a nucleic acid. Examples comprise use of a protein that binds to double-stranded nucleic acids (e.g., double-stranded DNA, double-stranded RNA, a double-stranded DNA/RNA hybrid) and/or a melting component (e.g., a protein or a nucleic acid) to dissociate a double-stranded nucleic acid (e.g., a region of a nucleic acid) to provide a single-stranded nucleic acid. In particular examples, the melting component comprises a dCas9/gRNA complex comprising a gRNA hybridized to the first region. In some examples, the melting component comprises a PAMmer, e.g., provided in trans to the target nucleic acid.

In some examples, the query probe hybridizes repeatedly to the second region with a kinetic rate constant $k_{off}$ that is greater than 0.1 min$^{-1}$ and/or a kinetic rate constant $k_{on}$ that is greater than 0.1 min$^{-1}$. In some examples, the query probe hybridizes repeatedly to the second region with a kinetic rate constant $k_{off}$ that is greater than 1 min$^{-1}$ and/or a kinetic rate constant $k_{on}$ that is greater than 1 min$^{-1}$. In some examples, the query probe is a fluorescently labeled nucleic acid that hybridizes repeatedly to the second region with a kinetic rate constant $k_{off}$ that is greater than 0.1 min$^{-1}$ and/or a kinetic rate constant $k_{on}$ that is greater than 0.1 min$^{-1}$. In some examples, the query probe is a fluorescently labeled nucleic acid that hybridizes repeatedly to the second region with a kinetic rate constant $k_{off}$ that is greater than 1 min$^{-1}$ and/or a kinetic rate constant $k_{on}$ that is greater than 1 min$^{-1}$.

In some examples, the melting component comprises a dCas9 that is immobilized to a substrate.

In some examples, data are analyzed, e.g., in some examples the fingerprint is detectable by a pattern recognition analysis.

Additional examples comprise a second melting component interacting with a third region of the target nucleic acid adjacent to the second region of the target nucleic acid. In some examples, the second melting component comprises a dCas9. In some examples, the second melting component comprises a single-stranded binding protein. In some examples, the second melting component is a protein and in some examples the second melting component is a nucleic acid. In particular examples, the second melting component comprises a dCas9/gRNA complex comprising a gRNA hybridized to the third region. In some examples, the melting component comprises a PAMmer, e.g., provided in trans to the target nucleic acid. In some examples, the first and second melting components bind approximately 5 to 15 nucleotides apart on the target nucleic acid, e.g., to provide access to the query region by a query probe.

The technology finds use in the detection, identification, and/or quantification of nucleic acids, e.g., in some examples, the target nucleic acid comprises a mutation, a single nucleotide polymorphism, or a modified base.

Additional examples provide a method for providing a detectable fingerprint of a double-stranded target nucleic acid in a sample, the method comprising immobilizing a double-stranded target nucleic acid to a discrete region of a solid support, said double-stranded target nucleic acid comprising a first region adjacent to a second region and said discrete region of said solid support comprising an immobilized melting component interacting with the first region; providing a query probe that binds repeatedly to the second region to provide a detectable fingerprint; and associating the detectable fingerprint with the double-stranded nucleic acid to identify the double-stranded nucleic acid. Some examples comprise analyzing data using pattern recognition or a similar analysis (e.g., machine learning, neural network, supervised and/or unsupervised learning, etc.) to produce or identify the detectable fingerprint of the double stranded nucleic acid.

In some methods, the melting component comprises a dCas9. In some methods, the melting component comprises a single-stranded binding protein. In some methods, the melting component is a protein and in some examples the melting component is a nucleic acid. In particular methods, the melting component comprises a dCas9/gRNA complex comprising a gRNA hybridized to the first region. In some methods, the melting component comprises a PAMmer, e.g., provided in trans to the target nucleic acid.

Additional examples comprise providing a second melting component that interacts with a third region of the target nucleic acid, said third target region adjacent to the second region. For example, some examples comprise providing a dCas9/gRNA complex comprising a gRNA complementary to a third region of the target nucleic acid adjacent to the second region of the target nucleic acid. Related examples comprise providing conditions sufficient for the melting component to provide the second region in a second stranded form, e.g., buffered pH conditions, temperature control, solution components (e.g., salts, counterions, cofactors, etc.), etc.

Some examples comprise detecting repeated binding of the query probe to the second region with a kinetic rate constant $k_{off}$ that is greater than 0.1 min$^{-1}$ and/or a kinetic rate constant $k_{on}$ that is greater than 0.1 min$^{-1}$. Some examples comprise detecting repeated binding of the query probe to the second region with a kinetic rate constant $k_{off}$ that is greater than 1 min$^{-1}$ and/or a kinetic rate constant $k_{on}$ that is greater than 1 min$^{-1}$. Some examples comprise detecting repeated binding of a fluorescently labeled nucleic acid to the second region with a kinetic rate constant $k_{off}$ that is greater than 0.1 min$^{-1}$ and/or a kinetic rate constant $k_{on}$ that is greater than 0.1 min$^{-1}$. Some examples comprise detecting repeated binding of a fluorescently labeled nucleic acid to the second region with a kinetic rate constant $k_{off}$ that is greater than 1 min$^{-1}$ and/or a kinetic rate constant $k_{on}$ that is greater than 1 min$^{-1}$.

Some examples comprise calculating an amount or concentration of the double-stranded target nucleic acid in the sample from the detectable fingerprint.

Examples of the technology provide a system for the detection of a double-stranded nucleic acid. In some examples, the system comprises a solid support comprising an immobilized melting component, a detectably labeled query probe that binds repeated to the double-stranded nucleic acid, a fluorescence detector, and a software component configured to perform pattern recognition analysis of query probe binding data. Systems may comprise compositions described herein and/or comprise components (e.g., a computer, processor, etc.) to perform methods as described herein.

The technology may find use in a method for calculating a predictor that a subject has or is at risk of having a cancer. For example, said methods may comprise determining the presence of a microRNA biomarker, determining the presence of a mutation, and determining the presence of a modified base in genomic DNA. The predictor may be a value calculated from variables associated with the presence of a microRNA biomarker, the presence of a mutation, and the presence of a modified base in genomic DNA.

Related examples provide a complex for detecting a target nucleic acid, the complex comprising a target nucleic acid comprising a first region adjacent to a second region; a detectably labeled capture probe hybridized to the first region; a query probe labeled with a quencher or fluorescent acceptor compatible with the label of the capture probe, wherein the query probe hybridizes repeatedly to the second region with a kinetic rate constant $k_{off}$ that is greater than 0.1 min-1 and/or a kinetic rate constant kon that is greater than 0.1 min-1.

## BRIEF DESCRIPTION OF THE DRAWINGS

These and other features, aspects, and advantages of the present technology will become better understood with regard to the following drawings:

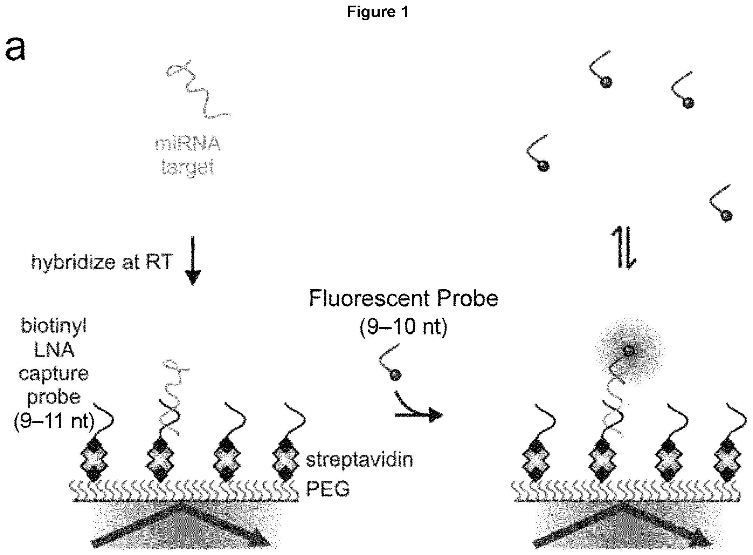

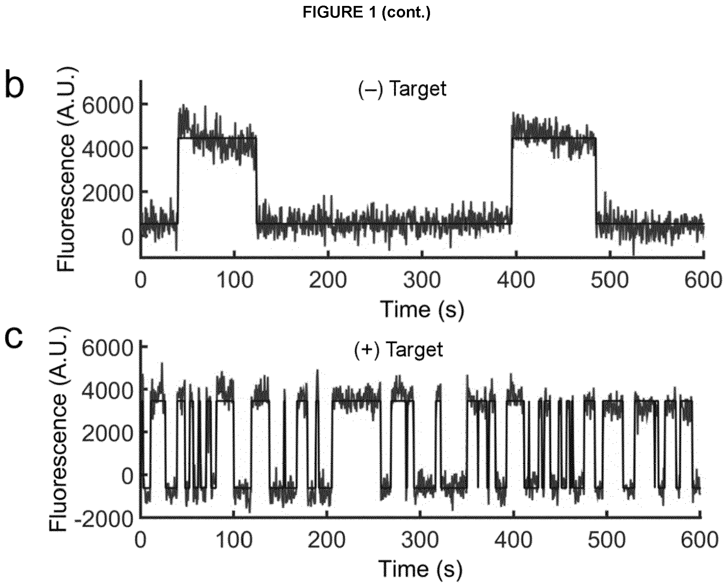

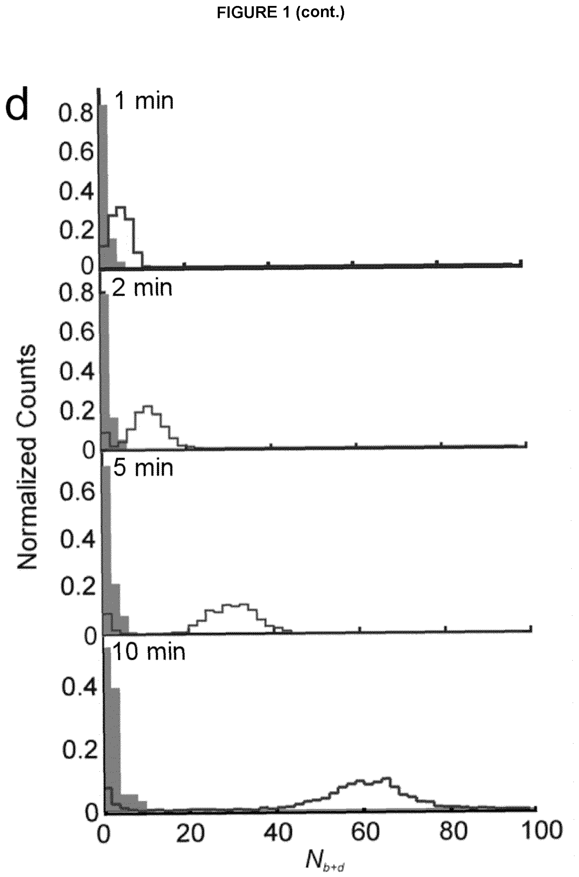

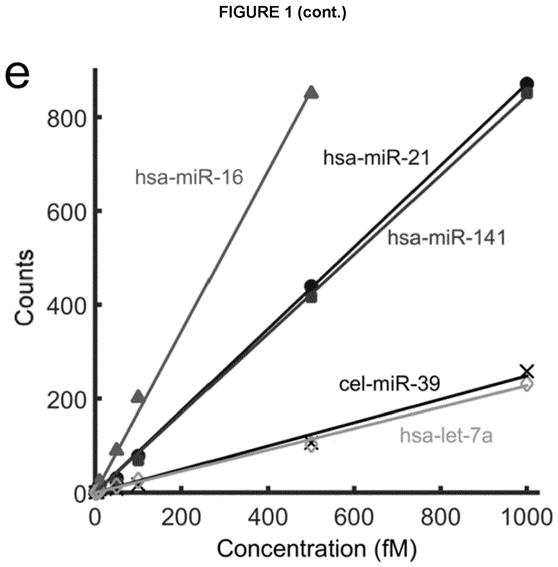

Figure 1 a is a schematic drawing of an example of the nucleic acid detection technology provided herein.
Figure 1 b shows exemplary SiMREPS data of fluorescently labeled query probes transiently associating non-specifically to a slide surface.

Figure 1 c shows exemplary SiMREPS data of fluorescently labeled query probes transiently binding to a target nucleic acid. The kinetic fingerprints of 1c and 1b are different, thus providing examples of the different kinetic signatures for specific and non-specific binding of query probes.

Figure 1 d is a series of histograms indicating the number of query probes counted to have a given number of intensity transitions ($N_{b+d}$) in the absence (thick gray bars) or presence (thin lines) of 1 pM target nucleic acid (e.g., a miR-141 microRNA). The four histograms plot data acquired with acquisition times of 1, 2, 5, and 10 minutes.

Figure 1 e shows plots of standard curves from SiMREPS assays of five miRNAs, yielding $R^2$ values > 0.99. The SiMREPS technology provides high-confidence detection of nucleic acids.

Figure 2 a is a plot showing that the fluorescent query probe for let-7a exhibits long lifetimes of binding to let-7a ($\tau_{on}$ = 23.3 $\pm$ 8.3 s) but much more transient binding to let-7c ($\tau_{on}$ = 4.7 $\pm$ 3.0 s) due to a single mismatch in the let-7c sequence relative to let-7a (underlined "G").

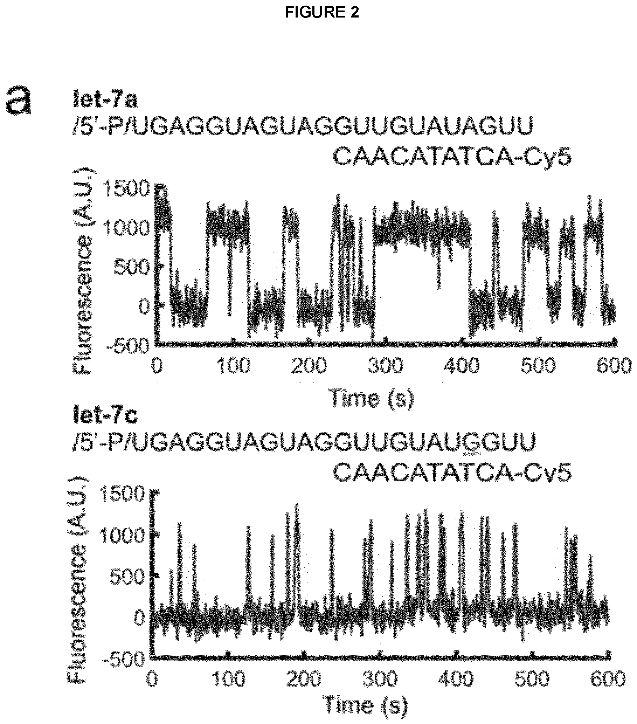

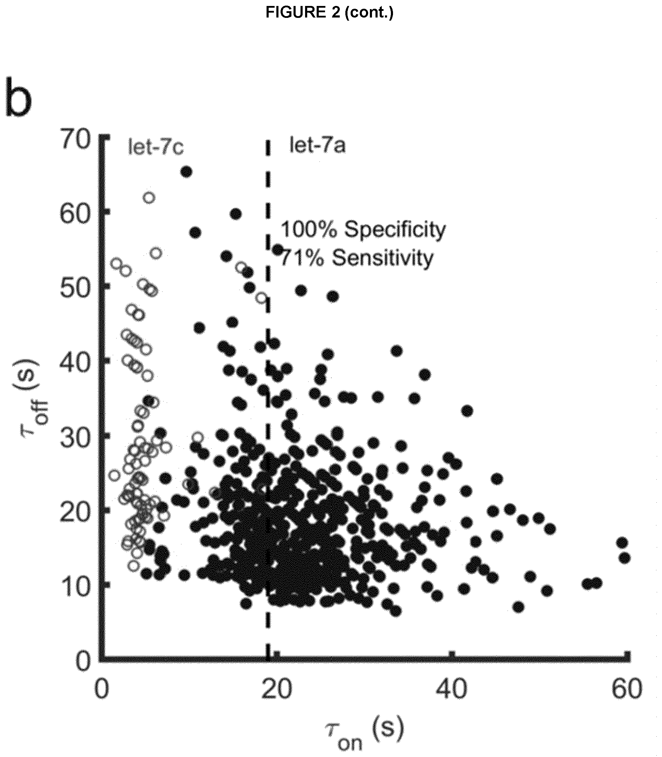

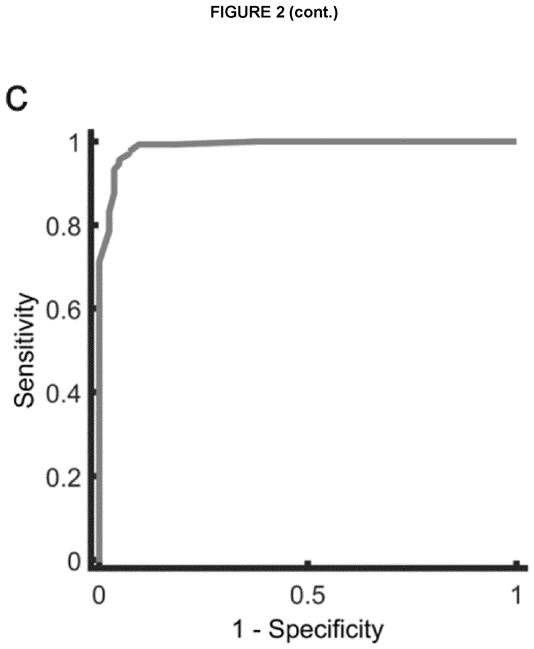

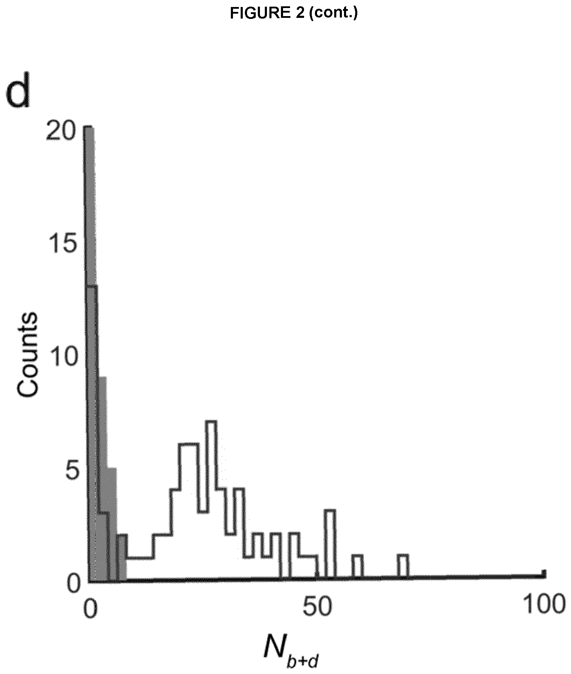

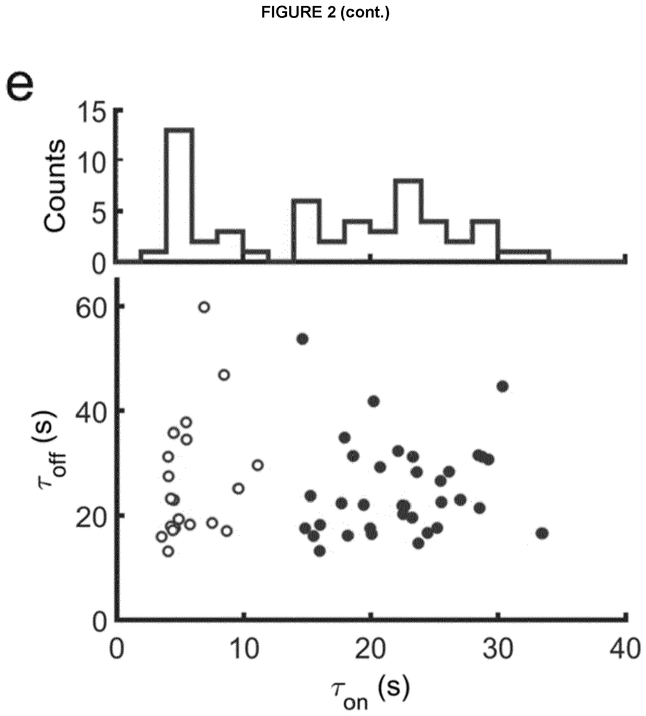

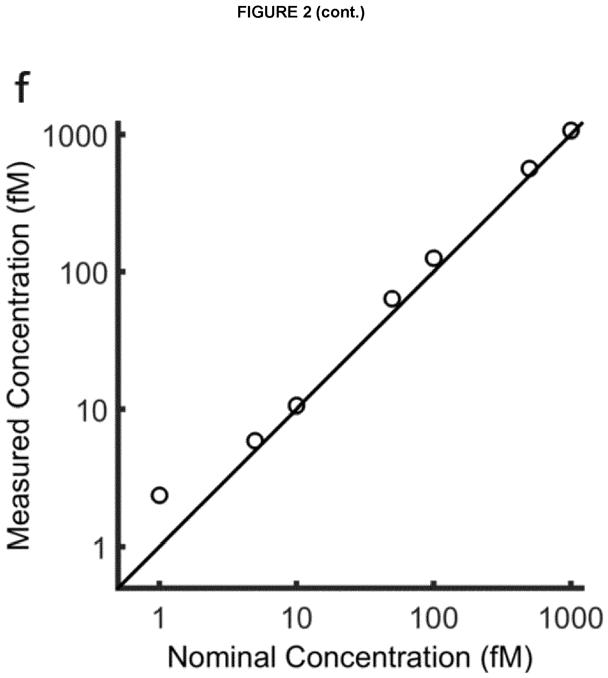

Figure 2 b is a dwell time analysis showing the high-confidence single-copy-level discrimination between let-7a (closed circles) and let-7c (open circles).

Figure 2 c is a receiver operating characteristic (ROC) plot constructed by varying the $\tau_{on}$ threshold for discriminating between let-7a and let-7c.

Figure 2 d is a $N_{b+d}$ histogram for the detection of let-7 in crude HeLa cell extract in the presence or absence of the miRCURY let-7 inhibitor. The $N_{b+d}$ histogram for endogenous hsa-let-7a showed a well-defined peak (thin line) that vanished in the presence of a let-7 inhibitor designed to bind and sequester let-7 family members (thick grey bars).

Figure 2 e shows the dwell times for molecules detected in crude HeLa extract using the fluorescent and capture probes for let-7a. The filled and open circles represent two clusters of target molecules classified by k-means clustering of $\tau_{on}$ values, consistent with the expected $\tau_{on}$ distributions for single-nucleotide mutants hsa-let-7a and hsa-let-7c.

Figure 2 f shows the quantification of synthetic miR-141 spiked into human serum.

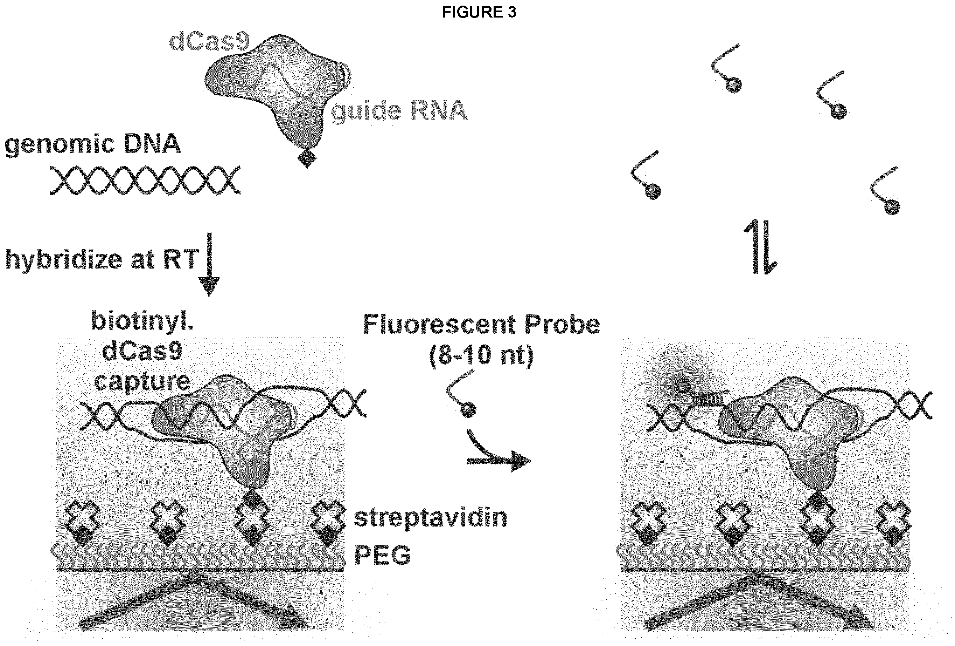

Figure 3 is a schematic diagram showing an example of the technology comprising a dCas9/gRNA. Genomic target DNA is briefly pre-treated with dCas9/gRNA. During this time, the guide RNA (gRNA) of the dCas9/gRNA complex hybridizes to the target nucleic acid (e.g., a genomic DNA) at a region adjacent to a query region (e.g., complementary to a query probe). The dCas9/gRNA melts a specific DNA sequence (e.g., the query region) in the target nucleic acid. After capture of the biotinylated dCas9 onto a slide surface (e.g., by biotin-avidin interaction), SiMREPS is used to detect binding of the query probe to the now accessible query region in the target nucleic acid, which is adjacent to the site of target nucleic acid hybridized to the gRNA.

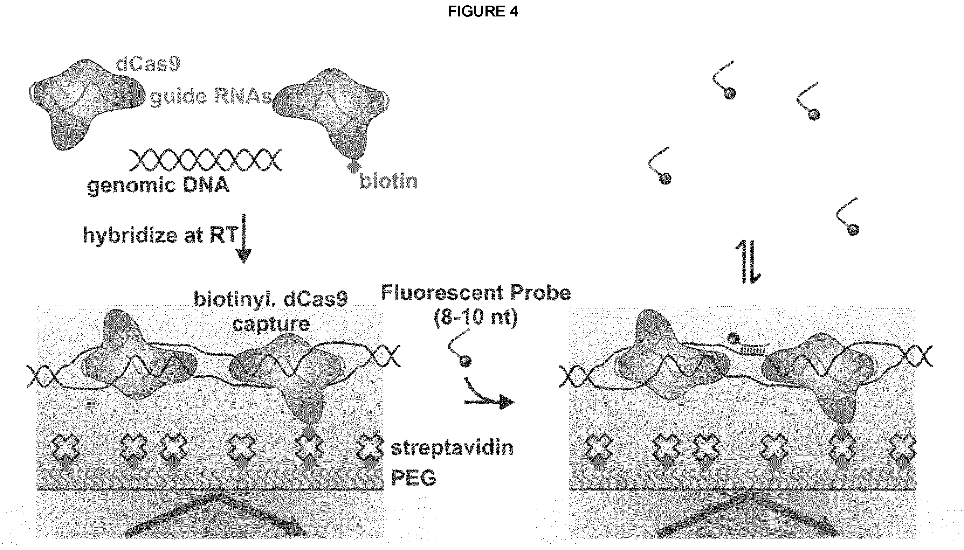

Figure 4 is a schematic diagram showing an example of the technology comprising hybridization of two flanking dCas9/gRNA complexes to a target nucleic acid. In some examples, hybridizing two dCas9/gRNA complexes to flank the query region further improves accessibility of the target nucleic acid (e.g., the query region) to the query probe for SiMREPS-based detection, and, in some examples, increases specificity. In the example shown in the figure, one dCas9 is biotinylated for capture onto the surface and the other dCas9 is not biotinylated, though in some examples the second dCas9 is modified, e.g., biotinylated.

Figure 5 a is a schematic showing an example of intramolecular SiMREPS probing in which the query and capture probes are linked on a contiguous oligonucleotide.

Figure 5 b is a schematic showing an example of intramolecular SiMREPS probing in which the non-contiguous query and capture probes are co-localized by an address oligonucleotide.

It is to be understood that the figures are not necessarily drawn to scale, nor are the objects in the figures necessarily drawn to scale in relationship to one another. The figures are depictions that are intended to bring clarity and understanding to various examples of apparatuses, systems, and methods disclosed herein. Wherever possible, the same reference numbers will be used throughout the drawings to refer to the same or like parts..

## DETAILED DESCRIPTION

The invention is set out in the appended claims. The embodiments of the description which do not fall within the scope of said claims are provided for illustrative purposes only and do not form part of the present invention. For the sake of clarity, the subject-matter described in the present specification not corresponding to the complex as defined in claims 1-6 and the methods as defined in claims 7-15 of the enclosed claim set are not part of the present invention. Provided herein is technology relating to detecting and identifying nucleic acids and particularly, but not exclusively, to compositions, methods, kits, and systems for detecting, identifying, and quantifying target nucleic acids with high confidence at single-molecule resolution

In this detailed description of the various examples, for purposes of explanation, numerous specific details are set forth to provide a thorough understanding of the examples disclosed. One skilled in the art will appreciate, however, that these various examples may be practiced with or without these specific details. In other instances, structures and devices are shown in block diagram form. Furthermore, one skilled in the art can readily appreciate that the specific sequences in which methods are presented and performed are illustrative and it is contemplated that the sequences can be varied.

Unless defined otherwise, all technical and scientific terms used herein have the same meaning as is commonly understood by one of ordinary skill in the art to which the various examples described herein belongs. When definitions of terms in references appear to differ from the definitions provided in the present teachings, the definition provided in the present teachings shall control. The section headings used herein are for organizational purposes only.

**Definitions**

To facilitate an understanding of the present technology, a number of terms and phrases are defined below. Additional definitions are set forth throughout the detailed description.

In addition, as used herein, the term "or" is an inclusive "or" operator and is equivalent to the term "and/or" unless the context clearly dictates otherwise. The term "based on" is not exclusive and allows for being based on additional factors not described, unless the context clearly dictates otherwise. In addition, throughout the specification, the meaning of "a", "an", and "the" include plural references. The meaning of "in" includes "in" and "on."

As used herein, a "nucleic acid" or a "nucleic acid sequence" refers to a polymer or oligomer of pyrimidine and/or purine bases, preferably cytosine, thymine, and uracil, and adenine and guanine, respectively (See Albert L. Lehninger, Principles of Biochemistry, at 793-800 (Worth Pub. 1982)). The present technology contemplates any deoxyribonucleotide, ribonucleotide, or peptide nucleic acid component, and any chemical variants thereof, such as methylated, hydroxymethylated, or glycosylated forms of these bases, and the like. The polymers or oligomers may be heterogenous or homogenous in composition, and may be isolated from naturally occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states. In some examples, a nucleic acid or nucleic acid sequence comprises other kinds of nucleic acid structures such as, for instance, a DNA/RNA helix, peptide nucleic acid (PNA), morpholino, locked nucleic acid (LNA), and/or a ribozyme. Hence, the term "nucleic acid" or "nucleic acid sequence" may also encompass a chain comprising non-natural nucleotides, modified nucleotides, and/or non- nucleotide building blocks that can exhibit the same function as natural nucleotides (e.g., "nucleotide analogs"); further, the term "nucleic acid sequence" as used herein refers to an oligonucleotide, nucleotide or polynucleotide, and fragments or portions thereof, and to DNA or RNA of genomic or synthetic origin, which may be single or double-stranded, and represent the sense or antisense strand.

The term "nucleotide analog" as used herein refers to modified or non-naturally occurring nucleotides including but not limited to analogs that have altered stacking interactions such as 7-deaza purines (i.e., 7-deaza-dATP and 7-deaza-dGTP); base analogs with alternative hydrogen bonding configurations (e.g., such as Iso-C and Iso-G and other non-standard base pairs described in U.S. Pat. No. 6,001,983 to S. Benner); non-hydrogen bonding analogs (e.g., non-polar, aromatic nucleoside analogs such as 2,4-difluorotoluene, described by B. A. Schweitzer and E. T. Kool, J. Org. Chem., 1994, 59, 7238-7242, B. A. Schweitzer and E. T. Kool, J. Am. Chem. Soc., 1995, 117, 1863-1872); "universal" bases such as 5-nitroindole and 3-nitropyrrole; and universal purines and pyrimidines (such as "K" and "P" nucleotides, respectively; P. Kong, et al., Nucleic Acids Res., 1989, 17, 10373-10383, P. Kong et al., Nucleic Acids Res., 1992, 20, 5149-5152). Nucleotide analogs include nucleotides having modification on the sugar moiety, such as dideoxy nucleotides and 2'-O-methyl nucleotides. Nucleotide analogs include modified forms of deoxyribonucleotides as well as ribonucleotides.

"Peptide nucleic acid" means a DNA mimic that incorporates a peptide-like polyamide backbone.

As used herein, the term "% sequence identity" refers to the percentage of nucleotides or nucleotide analogs in a nucleic acid sequence that is identical with the corresponding nucleotides in a reference sequence after aligning the two sequences and introducing gaps, if necessary, to achieve the maximum percent identity. Hence, in case a nucleic acid according to the technology is longer than a reference sequence, additional nucleotides in the nucleic acid, that do not align with the reference sequence, are not taken into account for determining sequence identity. Methods and computer programs for alignment are well known in the art, including blastn, Align 2, and FASTA.

The term "homology" and "homologous" refers to a degree of identity. There may be partial homology or complete homology. A partially homologous sequence is one that is less than 100% identical to another sequence.

The term "sequence variation" as used herein refers to differences in nucleic acid sequence between two nucleic acids. For example, a wild-type structural gene and a mutant form of this wild-type structural gene may vary in sequence by the presence of single base substitutions and/or deletions or insertions of one or more nucleotides. These two forms of the structural gene are said to vary in sequence from one another. A second mutant form of the structural gene may exist. This second mutant form is said to vary in sequence from both the wild-type gene and the first mutant form of the gene.

As used herein, the terms "complementary" or "complementarity" are used in reference to polynucleotides (e.g.,

a sequence of nucleotides such as an oligonucleotide or a target nucleic acid) related by the base-pairing rules. For example, for the sequence "5'-A-G-T-3'" is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids. Either term may also be used in reference to individual nucleotides, especially within the context of polynucleotides. For example, a particular nucleotide within an oligonucleotide may be noted for its complementarity, or lack thereof, to a nucleotide within another nucleic acid strand, in contrast or comparison to the complementarity between the rest of the oligonucleotide and the nucleic acid strand.

In some contexts, the term "complementarity" and related terms (e.g., "complementary", "complement") refers to the nucleotides of a nucleic acid sequence that can bind to another nucleic acid sequence through hydrogen bonds, e.g., nucleotides that are capable of base pairing, e.g., by Watson-Crick base pairing or other base pairing. Nucleotides that can form base pairs, e.g., that are complementary to one another, are the pairs: cytosine and guanine, thymine and adenine, adenine and uracil, and guanine and uracil. The percentage complementarity need not be calculated over the entire length of a nucleic acid sequence. The percentage of complementarity may be limited to a specific region of which the nucleic acid sequences that are base-paired, e.g., starting from a first base-paired nucleotide and ending at a last base-paired nucleotide. The complement of a nucleic acid sequence as used herein refers to an oligonucleotide which, when aligned with the nucleic acid sequence such that the 5' end of one sequence is paired with the 3' end of the other, is in "antiparallel association." Certain bases not commonly found in natural nucleic acids may be included in the nucleic acids of the present invention and include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs.

Thus, in some examples, "complementary" refers to a first nucleobase sequence that is at least 60%, 65%, 70%, 75%, 80%, 85%, 90%, 95%, 97%, 98%, or 99% identical to the complement of a second nucleobase sequence over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, or more nucleobases, or that the two sequences hybridize under stringent hybridization conditions. "Fully complementary" means each nucleobase of a first nucleic acid is capable of pairing with each nucleobase at a corresponding position in a second nucleic acid. For example, in certain examples, an oligonucleotide wherein each nucleobase has complementarity to a nucleic acid has a nucleobase sequence that is identical to the complement of the nucleic acid over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, or more nucleobases.

"Mismatch" means a nucleobase of a first nucleic acid that is not capable of pairing with a nucleobase at a corresponding position of a second nucleic acid.

As used herein, the term "hybridization" is used in reference to the pairing of complementary nucleic acids. Hybridization and the strength of hybridization (i.e., the strength of the association between the nucleic acids) is influenced by such factors as the degree of complementary between the nucleic acids, stringency of the conditions involved, and the $T_m$ of the formed hybrid. "Hybridization" methods involve the annealing of one nucleic acid to another, complementary nucleic acid, i.e., a nucleic acid having a complementary nucleotide sequence. The ability of two polymers of nucleic acid containing complementary sequences to find each other and anneal through base pairing interaction is a well-recognized phenomenon. The initial observations of the "hybridization" process by Marmur and Lane, Proc. Natl. Acad. Sci. USA 46:453 (1960) and Doty et al., Proc. Natl. Acad. Sci. USA 46:461 (1960) have been followed by the refinement of this process into an essential tool of modern biology.

As used herein, the term "$T_m$" is used in reference to the "melting temperature." The melting temperature is the temperature at which a population of double-stranded nucleic acid molecules becomes half dissociated into single strands. Several equations for calculating the $T_m$ of nucleic acids are well known in the art. As indicated by standard references, a simple estimate of the $T_m$ value may be calculated by the equation: $T_m = 81.5 + 0.41 * (\% G+C)$, when a nucleic acid is in aqueous solution at 1 M NaCl (see e.g., Anderson and Young, Quantitative Filter Hybridization, in Nucleic Acid Hybridization (1985). Other references (e.g., Allawi and SantaLucia, Biochemistry 36: 10581-94 (1997) include more sophisticated computations which account for structural, environmental, and sequence characteristics to calculate $T_m$. For example, in some examples these computations provide an improved estimate of $T_m$ for short nucleic acid probes and targets (e.g., as used in the examples).

As used herein, the term "melting" when used in reference to a nucleic acid refers to the dissociation of a double-stranded nucleic acid or region of a nucleic acid into a single-stranded nucleic acid or region of a nucleic acid.

As used herein, the term "melting component" refers to a substance, molecule (e.g., a biomolecule), or a complex of more than one molecule (e.g., a complex of more than one biomolecule) that interacts with a nucleic acid and melts it, e.g., dissociates double-stranded regions (e.g., secondary structure of a single-stranded nucleic acid, a duplex structure of DNA or of a RNA/DNA hybrid) to provide single-stranded regions, e.g., to provide access to query regions for binding of a query probe. A melting component can be a dCas9/gRNA complex (e.g., in some examples, comprising a biotinylated dCas9 and in some examples comprising a non-biotinylated dCas9). The technology is not limited, however, to melting components that comprise dCas9. The technology comprises use of any entity that provides access to a query region by a query probe and allows SiMREPS assay of a target nucleic acid.

As used herein, a "double-stranded nucleic acid" may be a portion of a nucleic acid, a region of a longer nucleic acid, or an entire nucleic acid. A "double-stranded nucleic acid" may be, e.g., without limitation, a double-stranded DNA, a double-stranded RNA, a double-stranded DNA/RNA hybrid, etc. A single-stranded nucleic acid having secondary structure (e.g., base-paired secondary structure) and/or higher order structure comprises a "double-stranded nucleic acid". For example, triplex structures are considered to be "double-stranded". In some examples, any base-paired nucleic acid is a "double-stranded nucleic acid"

As used herein, a "non-coding RNA" or "ncRNA" is a functional RNA molecule that is not translated into a protein. Less-frequently used synonyms are non-protein-coding RNA (npcRNA), non-messenger RNA (nmRNA), small non-messenger RNA (snmRNA), and functional RNA (fRNA). The term small RNA (sRNA) is often used for bacterial ncRNAs. The DNA sequence from which a non-coding RNA is transcribed as the end product is often called an RNA gene or a non-coding RNA gene. Non-coding RNA genes include highly abundant and functionally important RNAs such as transfer RNA (tRNA) and ribosomal RNA (rRNA), as well as RNAs such as snoRNAs, microRNAs, siRNAs, and piRNAs. The number of ncRNAs encoded within the human genome is unknown, however recent transcriptomic and bioinformatic studies suggest the existence of thousands of ncRNAs. Since most of the newly identified ncRNAs have not been validated for their function, it is possible that many are non-functional.

As used herein, the term "long non-coding RNA" or "lncRNA" or "long ncRNA" refers to a non-protein coding RNA longer than approximately 200 nucleotides. As used herein, the term is used to distinguish lncRNAs from small regulatory RNAs such as microRNAs (miRNAs), short interfering RNAs (siRNAs), Piwi-interacting RNAs (piRNAs), small nucleolar RNAs (snoRNAs), and other short RNAs.

As used herein, the term "miRNA" refers to microRNA. As used herein, the term "miRNA target sequence" refers to a miRNA that is to be detected (e.g., in the presence of other nucleic acids). In some examples, a miRNA target sequence is a variant of a miRNA.

The term "siRNAs" refers to short interfering RNAs. In some examples, siRNAs comprise a duplex, or double-stranded region, where each strand of the double-stranded region is about 18 to 25 nucleotides long; the double-stranded region can be as short as 16, and as long as 29, base pairs long, where the length is determined by the antisense strand. Often siRNAs contain from about two to four unpaired nucleotides at the 3' end of each strand. SiRNAs appear to function as key intermediates in triggering RNA interference in invertebrates and in vertebrates, and in triggering sequence-specific RNA degradation during posttranscriptional gene silencing in plants. At least one strand of the duplex or double-stranded region of a siRNA is substantially homologous to or substantially complementary to a target RNA molecule. The strand complementary to a target RNA molecule is the "antisense" strand; the strand homologous to the target RNA molecule is the "sense" strand and is also complementary to the siRNA antisense strand. One strand of the double-stranded region need not be the exact length of the opposite strand' thus, one strand may have at least one fewer nucleotides than the opposite complementary strand, resulting in a "bubble" or at least one unmatched base in the opposite strand. One strand of the double-stranded region need not be exactly complementary to the opposite strand; thus, the strand, preferably the sense strand, may have at least one mismatched base pair.

siRNAs may also contain additional sequences; non-limiting examples of such sequences include linking sequences, or loops, which connect the two strands of the duplex region. This form of siRNAs may be referred to "si-like RNA", "short hairpin siRNA" where the short refers to the duplex region of the siRNA, or "hairpin siRNA". Additional non-limiting examples of additional sequences present in siRNAs include stem and other folded structures. The additional sequences may or may not have known functions; non-limiting examples of such functions include increasing stability of an siRNA molecule, or providing a cellular destination signal.

"Pre-miRNA" or "pre-miR" means a non-coding RNA having a hairpin structure, which is the product of cleavage of a pri-miR by the double-stranded RNA-specific ribonuclease known as Drosha.

"Stem-loop sequence" means an RNA having a hairpin structure and containing a mature miRNA sequence. Pre-miRNA sequences and stem-loop sequences may overlap. Examples of stem-loop sequences are found in the miRNA database known as miRBase (available at the worldwide web at microma.sanger.ac.uk).

"Pri-miRNA" or "pri-miR" means a non-coding RNA having a hairpin structure that is a substrate for the double-stranded RNA-specific ribonuclease Drosha.

"miRNA precursor" means a transcript that originates from a genomic DNA and that comprises a non-coding, structured RNA comprising one or more miRNA sequences. For example, a miRNA precursor can be a pre-miRNA. In certain examples, a miRNA precursor is a pri-miRNA.

The term "gene" refers to a DNA sequence that comprises control and coding sequences necessary for the production of an RNA having a non-coding function (e.g., a ribosomal or transfer RNA), a polypeptide or a precursor. The RNA or polypeptide can be encoded by a full length coding sequence or by any portion of the coding sequence so long as the desired activity or function is retained.

The term "wild-type" refers to a gene or a gene product that has the characteristics of that gene or gene product when isolated from a naturally occurring source. A wild-type gene is that which is most frequently observed in a population and is thus arbitrarily designated the "normal" or "wild-type" form of the gene. In contrast, the term "modified," "mutant," or "polymorphic" refers to a gene or gene product that displays modifications in sequence and or functional properties (i.e., altered characteristics) when compared to the wild-type gene or gene product. It is noted that naturally-occurring mutants can be isolated; these are identified by the fact that they have altered characteristics when compared to the wild-type gene or gene product.

The term "oligonucleotide" as used herein is defined as a molecule comprising two or more deoxyribonucleotides or ribonucleotides, preferably at least 5 nucleotides, more preferably at least about 10 to 15 nucleotides and more preferably at least about 15 to 30 nucleotides. The exact size will depend on many factors, which in turn depend on the ultimate function or use of the oligonucleotide. The oligonucleotide may be generated in any manner, including chemical synthesis, DNA replication, reverse transcription, PCR, or a combination thereof.

Because mononucleotides are reacted to make oligonucleotides in a manner such that the 5' phosphate of one mononucleotide pentose ring is attached to the 3' oxygen of its neighbor in one direction via a phosphodiester linkage, an end of an oligonucleotide is referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide, also may be said to have 5' and 3' ends. A first region along a nucleic acid strand is said to be upstream of another region if the 3' end of the first region is before the 5' end of the second region when moving along a strand of nucleic acid in a 5' to 3' direction.

When two different, non-overlapping oligonucleotides anneal to different regions of the same linear complementary nucleic acid sequence, and the 3' end of one oligonucleotide points towards the 5' end of the other, the former may be called the "upstream" oligonucleotide and the latter the "downstream" oligonucleotide. Similarly, when two overlapping oligonucleotides are hybridized to the same linear complementary nucleic acid sequence, with the first oligonucleotide positioned such that its 5' end is upstream of the 5' end of the second oligonucleotide, and the 3' end of the first oligonucleotide is upstream of the 3' end of the second oligonucleotide, the first oligonucleotide may be called the "upstream" oligonucleotide and the second oligonucleotide may be called the "downstream" oligonucleotide.

As used herein, the terms "subject" and "patient" refer to any organisms including plants, microorganisms, and animals (e.g., mammals such as dogs, cats, livestock, and humans).

The term "sample" in the present specification and claims is used in its broadest sense. On the one hand it is meant to include a specimen or culture (e.g., microbiological cultures). On the other hand, it is meant to include both biological and environmental samples. A sample may include a specimen of synthetic origin.

As used herein, a "biological sample" refers to a sample of biological tissue or fluid. For instance, a biological sample may be a sample obtained from an animal (including a human); a fluid, solid, or tissue sample; as well as liquid and solid food and feed products and ingredients such as dairy items, vegetables, meat and meat byproducts, and waste. Biological samples may be obtained from all of the various families of domestic animals, as well as feral or wild animals, including, but not limited to, such animals as ungulates, bear, fish, lagomorphs, rodents, etc. Examples of biological samples include sections of tissues, blood, blood fractions, plasma, serum, urine, or samples from other peripheral sources or cell cultures, cell colonies, single cells, or a collection of single cells. Furthermore, a biological sample includes pools or mixtures of the above mentioned samples. A biological sample may be provided by removing a sample of cells from a subject, but can also be provided by using a previously isolated sample. For example, a tissue sample can be removed from a subject suspected of having a disease by conventional biopsy techniques. In some examples, a blood sample is taken from a subject. A biological sample from a patient means a sample from a subject suspected to be affected by a disease.

Environmental samples include environmental material such as surface matter, soil, water, and industrial samples, as well as samples obtained from food and dairy processing instruments, apparatus, equipment, utensils, disposable and non-disposable items. These examples are not to be construed as limiting the sample types applicable to the present invention.

The term "label" as used herein refers to any atom or molecule that can be used to provide a detectable (preferably quantifiable) effect, and that can be attached to a nucleic acid or protein. Labels include, but are not limited to, dyes (e.g., fluorescent dyes or moities); radiolabels such as $^{32}$P; binding moieties such as biotin; haptens such as digoxgenin; luminogenic, phosphorescent, or fluorogenic moieties; mass tags; and fluorescent dyes alone or in combination with moieties that can suppress or shift emission spectra by fluorescence resonance energy transfer (FRET). Labels may provide signals detectable by fluorescence, radioactivity, colorimetry, gravimetry, X-ray diffraction or absorption, magnetism, enzymatic activity, characteristics of mass or behavior affected by mass (e.g., MALDI time-of-flight mass spectrometry; fluorescence polarization), and the like. A label may be a charged moiety (positive or negative charge) or, alternatively, may be charge neutral. Labels can include or consist of nucleic acid or protein sequence, so long as the sequence comprising the label is detectable.

"Support" or "solid support", as used herein, refers to a matrix on or in which nucleic acid molecules, microparticles, and the like may be immobilized, e.g., to which they may be covalently or noncovalently attached or in or on which they may be partially or completely embedded so that they are largely or entirely prevented from diffusing freely or moving with respect to one another.

As used herein, "moiety" refers to one of two or more parts into which something may be divided, such as, for example, the various parts of an oligonucleotide, a molecule, a chemical group, a domain, a probe, etc.

As used herein, a "query probe" or "reader probe" is any entity (e.g., molecule, biomolecule, etc.) that recognizes a nucleic acid (e.g., binds to a nucleic acid, e.g., binds specifically to a nucleic acid). The query probe can be a protein that recognizes a nucleic acid (e.g., a nucleic acid binding protein, an antibody, antibody fragment, a transcription factor, or any other protein that binds to a particular sequence in a nucleic acid). In some other examples, the query probe is a nucleic acid (e.g., a DNA, an RNA, a nucleic acid comprising DNA and RNA, a nucleic acid comprising modified bases and/or modified linkages between bases; e.g., a nucleic acid as described hereinabove, a nucleic acid aptamer or any other nucleic acid that binds to a particular sequence in a nucleic acid). In some examples, the query probe is labeled, e.g., with a detectable label such as, e.g., a fluorescent moiety as described herein. In some examples, the query probe comprises more than one type of molecule (e.g., more than one of a protein, a nucleic acid, a chemical linker or a chemical moiety).

As used herein, a "capture probe" is any entity (e.g., molecule, biomolecule, etc.) that recognizes a nucleic acid (e.g., binds to a nucleic acid, e.g., binds specifically to a nucleic acid). The capture probe can be a protein that recognizes a nucleic acid (e.g., a nucleic acid binding protein, an antibody, a fragment of an antibody, a transcription factor, or any other protein that binds to a particular sequence in a nucleic acid). In some other examples, a capture probe is a nucleic acid (e.g., a DNA, an RNA, a nucleic acid comprising DNA and RNA, a nucleic acid comprising modified bases and/or modified linkages between bases; e.g., a nucleic acid as described hereinabove). In some examples, a capture probe is labeled, e.g., with a detectable label such as, e.g., a fluorescent moiety as described herein. In some examples, the capture probe comprises more than one type of molecule (e.g., more than one of a protein, a nucleic acid, a chemical linker or a chemical moiety).

### Description

Provided herein is a technique for the specific and ultrasensitive detection of single nucleic acids. As previously described (see, e.g., US 2016/0046988 A1), SiMREPS uses total internal reflection fluorescence (TIRF) microscopy, single-molecule visualization, and kinetic analysis of binding and release of fluorescently labeled probes to target molecules (see, e.g., Fig. 1 a). Target molecules are quantified by simple, amplification-free, direct counting upon kinetic fingerprint identification that provides for exquisite discrimination between single nucleotide variants, as demonstrated previously for the detection of microRNA (see, e.g., US 2016/0046988 A1). The technology provided herein provides for the detection of additional forms of nucleic acids, e.g., DNA, mutant DNA, methylated DNA, e.g., in an abundant wild-type background.

Existing techniques for nucleic acid detection utilize probes that form a thermodynamically stable complex with the target molecule, and are thus limited to weak and often unreliable thermodynamic discrimination against background signal or spurious targets. In contrast, the technology described herein utilizes probes that repeatedly bind to the target molecule at the query region and related methods to record the large number of independent binding events that occur for each observed target molecule. This repeated kinetic sampling provides a unique kinetic "fingerprint" for the target and provides for a highly specific and sensitive detection of nucleic acids. In some examples, the technology provides for the discrimination of two nucleic acid molecules that differ by as few as one nucleotide. In some examples, the technology provides for the discrimination of two nucleic acid molecules when one of the two nucleic acid molecules is present in a large excess (e.g., $10\times$; $100\times$; $1000\times$; $10,000\times$; or $1,000,000\times$ or more in excess). See, e.g., US 2016/0046988 A1.

In some examples, a labeled nucleic acid is detected, e.g., using an instrument to detect a signal produced by the label. For instance, some examples comprise use of a detectably labeled (e.g., fluorescently labeled) query probe and a detector of fluorescence emission such a fluorescent microscopy technique. The technology may find use as a diagnostic tool for identifying mutant or aberrantly expressed nucleic acid targets in biological samples. See, e.g., US 2016/0046988 A1.

This approach may involve the capture of unlabeled nucleic acids by a dCas9/gRNA complex linked to a solid support (e.g., glass or fused silica) and melting of a query region, followed by observation of the repeated, transient binding of a short detectably labeled (e.g., fluorescently labeled) nucleic acid (e.g., DNA) query probe to the query region.

The dCas9/gRNA complex may be attached or fixed to a solid support. In some examples, the dCas9/gRNA complex comprises a moiety that provides for the immobilization of the dCas9/gRNA complex to a solid support by interaction of the moiety with a second moiety attached to the solid support. The dCas9/gRNA complex may be fixed directly or indirectly to a solid support.

Any of a variety of materials may be used as a support for the dCas9/gRNA complex, e.g., matrices or particles made of nitrocellulose, nylon, glass, polyacrylate, mixed polymers, polystyrene, silane polypropylene, and magnetically attractable materials. A planar surface is a preferred support for imaging by microscopy as described herein. A dCas9/gRNA complex may be immobilized by linking it directly to the solid support, e.g., by using any of a variety of covalent linkages, chelation, or ionic interaction, or may be immobilized by linking it indirectly via one or more linkers joined to the support. In some examples, the linker is a nucleic acid; in some examples, the linker is a nucleic acid comprising one or more nucleotides that is/are not intended to hybridize (e.g., that do not hybridize) to the target nucleic acid capture region but that are intended to act as a spacer between the dCas9/gRNA complex and its solid support.

In some examples, the dCas9/gRNA complex comprises a biotin group (e.g., the dCas9/gRNA complex is biotinylated) and the solid support comprises a streptavidin group (e.g., attached to the solid support by a linker moiety, e.g., a polyethylene glycol (PEG) linker). The specific interaction of the biotin and streptavidin thus immobilizes the capture probe to the solid support (Figs. 1a, 3, and 4).

Various other chemical methods can be employed for the immobilization of a dCas9/gRNA complex to a solid support. An example of such a method is to use a combination of a maleimide group and a thiol (-SH) group. In this method, a thiol (-SH) group is bonded to a dCas9/gRNA complex, and the solid support comprises a maleimide group. Accordingly, the thiol group of the dCas9/gRNA complex reacts with the maleimide group on the solid support to form a covalent bond, whereby the dCas9/gRNA complex is immobilized. Introduction of the maleimide group can utilize a process of firstly allowing a reaction between a glass substrate and an aminosilane coupling agent and then introducing the maleimide group onto the glass substrate by a reaction of the amino group with an EMCS reagent (N-(6-maleimidocaproyloxy)succinimide, available from Dojindo). Introduction of the thiol group to a DNA can be carried out using 5'-Thiol-Modifier C6 (available from Glen Research) when the DNA is synthesized by an automatic DNA synthesizer.

Instead of the above-described combination of a thiol group and a maleimide group, a combination of, e.g., an epoxy group (on the solid support) and an amino group (dCas9/gRNA complex), is used in some examples as a combination of functional groups for immobilization. Surface treatments using various kinds of silane coupling agents are also effective. Other techniques for the attachment of proteins to solid supports and solid surfaces are known in the art.

## Poisson processes

Examples of the technology are related to single-molecule recognition by recording the characteristic kinetics of a probe (e.g., a query probe) binding to a target (e.g., a query region). In particular examples, this process is a Poisson process. A Poisson process is a continuous-time stochastic process that counts the number of events and the time that events (e.g., transient binding of a detectably labeled (e.g., fluorescent) query probe to an immobilized target) occur in a given time interval. The time interval between each pair of consecutive events has an exponential distribution and each interval is assumed to be independent of other intervals. The Poisson distribution is a discrete probability distribution that expresses the probability of a given number of the events occurring in the given time interval if these events occur with a known average rate and independently of the time since the last event. The Poisson distribution can also be used for the number of events in other specified intervals such as distance, area, or volume.

A Poisson distribution is a special case of the general binomial distribution where the number of trials $n$ is large, the probability of success $p$ is small, and the product $np = \lambda$ is moderate. In a Poisson process, the probability that a number of events $N$ is $j$ at any arbitrary time $t$ follows the Poisson probability distribution $P_j(t)$:

$$P_j(t) = \frac{e^{-\lambda t}(\lambda t)^j}{j!}, \; j = 0, 1, 2, \ldots \qquad (1).$$

That is, the number $N$ of events that occur up to time $t$ has a Poisson distribution with parameter $\lambda t$. Statistical and mathematical methods relevant to Poisson processes and Poisson distributions are known in the art. See, e.g., "Stochastic Processes (i): Poisson Processes and Markov Chains" in *Statistics for Biology and Health - Statistical Methods in Bioinformatics* (Ewans and Grant, eds.), Springer (New York, 2001), page 129 et seq.. Software packages such as Matlab and R may be used to perform mathematical and statistical methods associated with Poisson processes, probabilities, and distributions.

## Kinetics of detection

Particular examples of the technology are related to detecting a nucleic acid by analyzing the kinetics of the interaction of a query probe with a query region of a target nucleic acid to be detected. For the interaction of a query probe Q (e.g., at an equilibrium concentration [Q]) with a target nucleic acid T (e.g., at an equilibrium concentration [T]), the kinetic rate constant $k_{on}$ describes the time-dependent formation of the complex QT comprising the query probe Q hybridized to the query region of the target nucleic acid T. In particular examples, while the formation of the QT complex is associated with a second order rate constant that is dependent on the concentration of query probe and has units of $M^{-1}min^{-1}$ (or the like), the formation of the QT complex is sufficiently described by a $k_{on}$ that is a pseudo-first order rate constant associated with the formation of the QT complex. Thus, as used herein, $k_{on}$ is an apparent ("pseudo") first-order rate constant.

Likewise, the kinetic rate constant $k_{off}$ describes the time-dependent dissociation of the complex QT into the query probe Q and the target nucleic acid T. Kinetic rates are typically provided herein in units of $min^{-1}$ or $s^{-1}$. The "dwell time" of the query probe Q in the bound state ($\tau_{on}$) is the time interval (e.g., length of time) that the probe Q is hybridized to the query region of the target nucleic acid T during each instance of query probe Q binding to the query region of the target nucleic acid T to form the QT complex. The "dwell time" of the query probe Q in the unbound state ($\tau_{off}$) is the time interval (e.g., length of time) that the probe Q is not hybridized to the query region of the target nucleic acid T between each instance of query probe Q binding to the query region of the target nucleic acid T to form the QT complex (e.g., the time the query probe Q is dissociated from the target nucleic acid T between successive binding events of the query probe Q to the target nucleic acid T). Dwell times may be provided as averages or weighted averages integrating over numerous binding and non-binding events.

Further, in some examples, the repeated, stochastic binding of query probes (e.g., detectably labeled query probes (e.g., fluorescent probes), e.g., nucleic acid probes such as DNA or RNA probes) to immobilized targets is modeled as a Poisson process occurring with constant probability per unit time and in which the standard deviation in the number of binding and dissociation events per unit time ($N_{b+d}$) increases as ($N_{b+d}$)$^{1/2}$. Thus, the statistical noise becomes a smaller fraction of Nb+d as the observation time is increased. Accordingly, the observation is lengthened as needed in some examples to achieve discrimination between target and off-target binding. And, as the acquisition time is increased, the signal and background peaks in the Nb+d histogram become increasingly separated and the width of the signal distribution increases as the square root of Nb+d, consistent with kinetic Monte Carlo simulations. An acquisition time of approximately 10 minutes (e.g., approximately 1 to 100 minutes, e.g., 1, 2, 3, 4, 5, 6, 7, 8, 9, 10, 11, 12, 13, 14, 15, 16, 17, 18, 19, 20, 30, 40, 50, 60, 70, 80, 90, or 100 minutes) yields sufficient (e.g., complete) separation of the signal from background distributions of Nb+d, providing for substantially background-free quantification of the target. See, e.g., US 2016/0046988 A1.

Further, in some examples the probe length is chosen to provide sufficient separation of signal and background peaks on convenient experimental time scales. In particular, the kinetics of query probe exchange are related to the number of complementary bases between the query probe and target nucleic acid. For instance, in some examples, the interaction of a short DNA query probe with its complement increases as an approximately exponential function of the number of base pairs formed, while the rate constant of binding is affected only weakly for interactions comprising at least 6 to 7 base pairs. Thus, varying query probe length provides for tuning the kinetic behavior to improve discrimination of query probe binding events to the target from background binding. In particular, a query (e.g., fluorescent) probe length of 9 nt to 10 nt (providing theoretical $T_m$ values of 17.5°C to 25°C) yields rapid target binding that is distinguished from background signal, as displayed in histograms of intensity transitions per candidate molecule in the presence and absence of target. See, e.g., US 2016/0046988 A1. Further, in some examples the kinetics of binding and dissociation are more closely correlated to probe length than to the melting temperature of the duplex. While some examples comprise use of a probe having a length of 9 to 10 nt, the technology is not limited by this length. Indeed, use of probes longer or shorter than 9 to 10 nt is contemplated by the technology, e.g., as discussed throughout.

## Detection of double-stranded nucleic acids

Examples of the technology provide for the detection of double-stranded nucleic acids. Some examples provide compositions, reaction mixtures, and complexes comprising a plurality of molecules for detecting one or more nucleic acids. Some examples of compositions, reaction mixtures, and complexes comprise a nucleic acid (e.g., a target nucleic acid) that is to be detected, identified, quantified, and/or characterized; a solid substrate comprising a dCas9/gRNA complex linked to a solid surface that binds one or more regions of the target with high specificity; and a detectably labeled (e.g., fluorescent) query probe. Some examples further comprise a protospacer adjacent motif (PAM) DNA oligonucleotide.

The SiMREPS technology exploits the direct binding of a short (6-12-nucleotide) fluorescently labeled DNA probe to an unlabeled nucleic acid target (e.g., miRNA) immobilized on a glass surface (Fig. 1 a) (see, e.g., US 2016/0046988 A1). Using TIRF microscopy (Walter et al (2008) "Do-it-yourself guide: how to use the modern single-molecule toolkit" Nat Methods 5: 475-89), both non-specific surface binding (Fig. 1 b) and specific binding to the immobilized target (Fig. 1 c) are detected. However, equilibrium binding of the probe to target yields a distinctive kinetic signature, or fingerprint, that can achieve ultra-high discrimination against background binding (compare Fig. 1 b with Fig. 1 c). Since the transient binding of probes to an immobilized target resembles a Poisson process, the standard deviation in the number of binding and dissociation events ($N_{b+d}$) increases as $\sqrt{N_{b+d}}$ As experimental acquisition time is increased, the signal and background peaks in histograms of Nb+d are progressively better-resolved (Fig. 1 d) (see, e.g., Johnson-Bucket al. (2015) "Kinetic fingerprinting to identify and count single nucleic acids" Nat Biotechnol 33: 730-2), allowing for arbitrarily high discrimination between target and off-target binding.

SiMREPS finds use in quantifying RNA (see, e.g., US 2016/0046988 A1). In particular, previous experiments quantified four human miRNAs that are dysregulated in cancer and other diseases. Discrimination (e.g., specificity = 1) was achieved for all target-probe pairs and standard curves showed linear dependence on target concentration (Fig. 1 e). A single fluorescent probe discriminates with highest specificity between two microRNAs differing by a single nucleotide (Fig. 2 a-c). SiMREPS detects targets in complex biological matrices. (Fig. 2 d, e). Also the prostate cancer biomarker hsa-miR-1418 was detected in a serum sample after spiking-in varying target concentrations. The measured concentration was strongly correlated with the nominal spiked-in concentration (Fig. 2 f, R >0.999, slope = 1.07).

One challenge in applying the SiMREPS technology to the detection of double-stranded nucleic acids is that the transient binding of a query probe to the target nucleic acid (e.g., at the query region) competes with association of the complementary strand to the query region (e.g., dsDNA) at the target locus. Accordingly, provided herein is a technology in which SiMREPS finds use in detecting double-stranded DNA. To overcome this challenge, some examples of the technology comprise use of a catalytically inactive ("dead") dCas9 enzyme loaded with a specific guide-RNA (gRNA) to melt dsDNA structure locally in a sequence-specific fashion, providing access for the SiMREPS probe (Fig. 3). Related examples comprise use of a protein that binds to double-stranded nucleic acids (e.g., double-stranded DNA, double-stranded RNA, a double-stranded DNA/RNA hybrid) and/or a melting component (e.g., a protein or a nucleic acid) to dissociate a double-stranded nucleic acid (e.g., a region of a nucleic acid) to provide a single-stranded nucleic acid.

### dCas9/gRNA complexes

The technology comprises use of a sequence-specific nucleic acid binding component (e.g., molecule, biomolecule, or complex of one or more molecules and/or biomolecules) to immobilize nucleic acids and/or convert double-stranded nucleic acids (e.g., regions of double-stranded nucleic acids) to single-stranded regions. The sequence-specific nucleic acid binding component may comprise an enzymatically inactive, or "dead", Cas9 protein ("dCas9") and a guide RNA ("gRNA"). While nucleic acid-binding molecules such as the clustered regularly interspaced short palindromic repeats (CRISPR) and CRISPR-associated proteins (Cas) (CRISPR/Cas) system have been used extensively for genome editing in cells of various types and species, recombinant and engineered nucleic acid-binding proteins find use in the present technology to melt double-stranded nucleic acids and provide single-stranded nucleic acids for probe binding.

The Cas9 protein was discovered as a component of the bacterial adaptive immune system (see, e.g., Barrangou et al. (2007) "CRISPR provides acquired resistance against viruses in prokaryotes" Science 315: 1709-1712). Cas9 is an RNA-guided endonuclease that targets and destroys foreign DNA in bacteria using RNA:DNA base-pairing between the gRNA and foreign DNA to provide sequence specificity. Recently, Cas9/gRNA complexes have found use in genome editing (see, e.g., Doudna et al. (2014) "The new frontier of genome engineering with CRISPR-Cas9" Science 346: 6213).

Accordingly, some Cas9/RNA complexes comprise two RNA molecules: (1) a CRISPR RNA (crRNA), possessing a nucleotide sequence complementary to the target nucleotide sequence; and (2) a trans-activating crRNA (tracrRNA). In this mode, Cas9 functions as an RNA-guided nuclease that uses both the crRNA and tracrRNA to recognize and cleave a target sequence. Recently, a single chimeric guide RNA (sgRNA) mimicking the structure of the annealed crRNA/tracrRNA has become more widely used than crRNA/tracrRNA because the gRNA approach provides a simplified system with only two components (e.g., the Cas9 and the sgRNA). Thus, sequence-specific binding to a nucleic acid can be guided by a natural dual-RNA complex (e.g., comprising a crRNA, a tracrRNA, and Cas9) or a chimeric single-guide RNA (e.g., a sgRNA and Cas9). (see, e.g., Jinek et al. (2012) "A Programmable Dual-RNA-Guided DNA Endonuclease in Adaptive Bacterial Immunity" Science 337:816-821).

As used herein, the targeting region of a crRNA (2-RNA system) or a sgRNA (single guide system) is referred to as the "guide RNA" (gRNA). In some examples, the gRNA comprises, consists of, or essentially consists of 10 to 50 bases, e.g., 15 to 40 bases, e.g., 15 to 30 bases, e.g., 15 to 25 bases (e.g., 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 bases). Methods are known in the art for determining the length of the gRNA that provides the most efficient target recognition for a Cas9. See, e.g., Lee et al. (2016) "The *Neisseria meningitidis* CRISPR-Cas9 System Enables Specific Genome Editing in Mammalian Cells" Molecular Therapy, 19 January 2016; doi:10.1038/mt.

Accordingly, in some examples the gRNA is a short synthetic RNA comprising a "scaffold" sequence for Cas9-binding and a user-defined approximately 20-nucleotide "targeting" sequence that is complementary to the nucleic acid target.

DNA targeting specificity may be determined by two factors: 1) a DNA sequence matching the gRNA targeting sequence and a protospacer adjacent motif (PAM) directly downstream of the target sequence. Some Cas9/gRNA complexes recognize a DNA sequence comprising a protospacer adjacent motif (PAM) sequence and the adjacent approximately 20 bases complementary to the gRNA. Canonical PAM sequences are NGG or NAG for Cas9 from *Streptococcus pyogenes* and NNNNGATT for the Cas9 from *Neisseria meningitidis.* Following DNA recognition by hybridization of the gRNA to the DNA target sequence, Cas9 cleaves the DNA sequence via an intrinsic nuclease activity. For genome editing and other purposes, the CRISPR/Cas system from *S. pyogenes* has been used most often. Using this system, one can target a given target nucleic acid (e.g., for editing or other manipulation) by designing a gRNA having nucleotide sequence complementary to an approximately 20-base DNA sequence 5'-adjacent to the PAM. Methods are known in the art for determining the PAM sequence that provides the most efficient target recognition for a Cas9. See, e.g., Zhang et al. (2013) "Processing-independent CRISPR RNAs limit natural transformation in Neisseria meningitidis" Molecular Cell 50: 488-503; Lee et al., supra.

The present technology comprises use of a catalytically inactive form of Cas9 ("dead Cas9" or "dCas9"), in which point mutations are introduced that disable the nuclease activity. In some examples, the dCas9 protein is from *S. pyogenes.* In some examples, the dCas9 protein comprises mutations at, e.g., D10, E762, H983, and/or D986; and at H840 and/or N863, e.g., at D10 and H840, e.g., D10A or D10N and H840A or H840N or H840Y. In some examples, the dCas9 is provided as a fusion protein comprising a functional domain for attaching the dCas9 to a solid surface (e.g., an epitope tag, linker peptide, etc.)

The dCas9/gRNA complex binds to a target nucleic acid with a sequence specificity provided by the gRNA, but does not cleave the nucleic acid. In this form, the dCas9/gRNA "melts" the target sequence to provide single-stranded regions of the target nucleic acid in a sequence-specific manner (see, e.g., Qi et al. (2013) "Repurposing CRISPR as an RNA-guided platform for sequence-specific control of gene expression" Cell 152(5): 1173-83).

Furthermore, while the Cas9/gRNA system and dCas9/gRNA system initially targeted sequences adjacent to a PAM, the dCas9/gRNA system as used herein has been engineered to target any nucleotide sequence for binding. Also, Cas9 and dCas9 orthologs encoded by compact genes (e.g., Cas9 from *Staphylococcus aureus)* are known (see, e.g., Ran et al. (2015) "In vivo genome editing using Staphylococcus aureus Cas9" Nature 520: 186-191), which improves the cloning and manipulation of the Cas9 components in vitro.

A number of bacteria express Cas9 protein variants. The Cas9 from Streptococcus pyogenes is presently the most commonly used; some of the other Cas9 proteins have high levels of sequence identity with the S. pyogenes Cas9 and use the same guide RNAs. Others are more diverse, use different gRNAs, and recognize different PAM sequences as well (the 2-5 nucleotide sequence specified by the protein which is adjacent to the sequence specified by the RNA). Chylinski et al. classified Cas9 proteins from a large group of bacteria (RNA Biology 10:5, 1-12; 2013), and a large number of Cas9 proteins are listed in supplementary FIG. 1 and supplementary table 1 thereof. Additional Cas9 proteins are described in Esvelt et al., Nat Methods. 2013 November; 10(11):1116-21 and Fonfara et al., "Phylogeny of Cas9 determines functional exchangeability of dual-RNA and Cas9 among orthologous type II CRISPR-Cas systems." Nucleic Acids Res. 2013 Nov. 22. [Epub ahead of print] doi:10.1093/nar/gkt1074.

Cas9, and thus dCas9, molecules of a variety of species find use in the technology described herein. While the *S. pyogenes* and *S. thermophilus* Cas9 molecules are widely used, Cas9 molecules of, derived from, or based on the Cas9 proteins of other species listed herein find use in examples of the technology. Accordingly, the technology provides for the replacement of *S. pyogenes* and *S. thermophilus* Cas9 and dCas9 molecules with Cas9 and dCas9 molecules from the other species can replace them, e.g.:

| GenBank Acc No. | Bacterium |
| --- | --- |
| 303229466 | Veillonella atypica ACS-134-V-Col7a |
| 34762592 | Fusobacterium nucleatum subsp. vincentii |
| 374307738 | Filifactor alocis ATCC 35896 |
| 320528778 | Solobacterium moorei F0204 |
| 291520705 | Coprococcus catus GD-7 |
| 42525843 | Treponema denticola ATCC 35405 |
| 304438954 | Peptoniphilus duerdenii ATCC BAA-1640 |
| 224543312 | Catenibacterium mitsuokai DSM 15897 |
| 24379809 | Streptococcus mutans UA159 |
| 15675041 | Streptococcus pyogenes SF370 |
| 16801805 | Listeria innocua Clip11262 |
| 116628213 | Streptococcus thermophilus LMD-9 |

EP 3 800 249 B1

(continued)

| GenBank Acc No. | Bacterium |
| --- | --- |
| 323463801 | Staphylococcus pseudintermedius ED99 |
| 352684361 | Acidaminococcus intestini RyC-MR95 |
| 302336020 | Olsenella uli DSM 7084 |
| 366983953 | Oenococcus kitaharae DSM 17330 |
| 310286728 | Bifidobacterium bifidum S17 |
| 258509199 | Lactobacillus rhamnosus GG |
| 300361537 | Lactobacillus gasseri JV-V03 |
| 169823755 | Finegoldia magna ATCC 29328 |
| 47458868 | Mycoplasma mobile 163K |
| 284931710 | Mycoplasma gallisepticum str. F |
| 363542550 | Mycoplasma ovipneumoniae SC01 |
| 384393286 | Mycoplasma canis PG 14 |
| 71894592 | Mycoplasma synoviae 53 |
| 238924075 | Eubacterium rectale ATCC 33656 |
| 116627542 | Streptococcus thermophilus LMD-9 |
| 315149830 | Enterococcus faecalis TX0012 |
| 315659848 | Staphylococcus lugdunensis M23590 |
| 160915782 | Eubacterium dolichum DSM 3991 |
| 336393381 | Lactobacillus coryniformis subsp. torquens |
| 310780384 | Ilyobacter polytropus DSM 2926 |
| 325677756 | Ruminococcus albus 8 |
| 187736489 | Akkermansia muciniphila ATCC BAA-835 |
| 117929158 | Acidothermus cellulolyticus 11B |
| 189440764 | Bifidobacterium longum DJO10A |
| 283456135 | Bifidobacterium dentium Bd1 |
| 38232678 | Corynebacterium diphtheriae NCTC 13129 |
| 187250660 | Elusimicrobium minutum Pei191 |
| 319957206 | Nitratifractor salsuginis DSM 16511 |
| 325972003 | Sphaerochaeta globus str. Buddy |
| 261414553 | Fibrobacter succinogenes subsp. succinogenes |
| 60683389 | Bacteroides fragilis NCTC 9343 |
| 256819408 | Capnocytophaga ochracea DSM 7271 |
| 90425961 | Rhodopseudomonas palustris BisB18 |
| 373501184 | Prevotella micans F0438 |
| 294674019 | Prevotella ruminicola 23 |
| 365959402 | Flavobacterium columnare ATCC 49512 |
| 312879015 | Aminomonas paucivorans DSM 12260 |
| 83591793 | Rhodospirillum rubrum ATCC 11170 |
| 294086111 | Candidatus Puniceispirillum marinum IMCC1322 |
| 121608211 | Verminephrobacter eiseniae EF01-2 |
| 344171927 | Ralstonia syzygii R24 |
| 159042956 | Dinoroseobacter shibae DFL 12 |
| 288957741 | Azospirillum sp- B510 |
| 92109262 | Nitrobacter hamburgensis X14 |
| 148255343 | Bradyrhizobium sp- BTAi1 |
| 34557790 | Wolinella succinogenes DSM 1740 |
| 218563121 | Campylobacter jejuni subsp. jejuni |
| 291276265 | Helicobacter mustelae 12198 |
| 229113166 | Bacillus cereus Rock1-15 |
| 222109285 | Acidovorax ebreus TPSY |

(continued)

| GenBank Acc No. | Bacterium |
|---|---|
| 189485225 | uncultured Termite group 1 |
| 182624245 | Clostridium perfringens D str. |
| 220930482 | Clostridium cellulolyticum H10 |
| 154250555 | Parvibaculum lavamentivorans DS-1 |
| 257413184 | Roseburia intestinalis L1-82 |
| 218767588 | Neisseria meningitidis Z2491 |
| 15602992 | Pasteurella multocida subsp. multocida |
| 319941583 | Sutterella wadsworthensis 3 1 |
| 254447899 | gamma proteobacterium HTCC5015 |
| 54296138 | Legionella pneumophila str. Paris |
| 331001027 | Parasutterella excrementihominis YIT 11859 |
| 34557932 | Wolinella succinogenes DSM 1740 |
| 118497352 | Francisella novicida U112 |

The technology described herein encompasses the use of a dCas9 derived from any Cas9 protein (e.g., as listed above) and their corresponding guide RNAs or other guide RNAs that are compatible. The Cas9 from *Streptococcus thermophilus* LMD-9 CRISPR1 system has been shown to function in human cells (see, e.g., Cong et al. (2013) Science 339: 819). Additionally, Jinek showed in vitro that Cas9 orthologs from *S. thermophilus* and *L. innocua,* can be guided by a dual *S. pyogenes* gRNA to cleave target plasmid DNA.

The present technology may comprise the Cas9 protein from *S. pyogenes,* either as encoded in bacteria or codon-optimized for expression in mammalian cells, containing mutations at D10, E762, H983, or D986 and H840 or N863, e.g., D10A/D10N and H840A/H840N/H840Y, to render the nuclease portion of the protein catalytically inactive; substitutions at these positions are, in some examples, alanine (Nishimasu (2014) Cell 156: 935-949) or, in some examples, other residues, e.g., glutamine, asparagine, tyrosine, serine, or aspartate, e.g., E762Q, H983N, H983Y, D986N, N863D, N863S, or N863H. The sequence of one *S. pyogenes* dCas9 protein that finds use in the technology provided herein is described in US20160010076.

For example, the dCas9 used herein is at least about 50% identical to the sequence of *S. pyogenes* Cas9, e.g., at least 50% identical to the following sequence of dCas9 comprising the D10A and H840A substitutions (SEQ ID NO: 1).

```
Met Asp Lys Lys Tyr Ser Ile Gly Leu Ala Ile Gly Thr Asn Ser Val
1               5               10              15
Gly Trp Ala Val Ile Thr Asp Glu Tyr Lys Val Pro Ser Lys Lys Phe
            20              25              30
Lys Val Leu Gly Asn Thr Asp Arg His Ser Ile Lys Lys Asn Leu Ile
        35              40              45
Gly Ala Leu Leu Phe Asp Ser Gly Glu Thr Ala Glu Ala Thr Arg Leu
    50              55              60
Lys Arg Thr Ala Arg Arg Arg Tyr Thr Arg Arg Lys Asn Arg Ile Cys
65              70              75              80
Tyr Leu Gln Glu Ile Phe Ser Asn Glu Met Ala Lys Val Asp Asp Ser
            85              90              95
Phe Phe His Arg Leu Glu Glu Ser Phe Leu Val Glu Glu Asp Lys Lys
        100             105             110
His Glu Arg His Pro Ile Phe Gly Asn Ile Val Asp Glu Val Ala Tyr
        115             120             125
His Glu Lys Tyr Pro Thr Ile Tyr His Leu Arg Lys Lys Leu Val Asp
    130             135             140
Ser Thr Asp Lys Ala Asp Leu Arg Leu Ile Tyr Leu Ala Leu Ala His
145             150             155             160
Met Ile Lys Phe Arg Gly His Phe Leu Ile Glu Gly Asp Leu Asn Pro
```

```
                            165                  170                  175
        Asp Asn Ser Asp Val Asp Lys Leu Phe Ile Gln Leu Val Gln Thr Tyr
                    180                  185                  190
        Asn Gln Leu Phe Glu Glu Asn Pro Ile Asn Ala Ser Gly Val Asp Ala
                    195                  200                  205
        Lys Ala Ile Leu Ser Ala Arg Leu Ser Lys Ser Arg Arg Leu Glu Asn
                    210                  215                  220
        Leu Ile Ala Gln Leu Pro Gly Glu Lys Lys Asn Gly Leu Phe Gly Asn
        225                  230                  235                  240
        Leu Ile Ala Leu Ser Leu Gly Leu Thr Pro Asn Phe Lys Ser Asn Phe
                    245                  250                  255
        Asp Leu Ala Glu Asp Ala Lys Leu Gln Leu Ser Lys Asp Thr Tyr Asp
                    260                  265                  270
        Asp Asp Leu Asp Asn Leu Leu Ala Gln Ile Gly Asp Gln Tyr Ala Asp
                    275                  280                  285
        Leu Phe Leu Ala Ala Lys Asn Leu Ser Asp Ala Ile Leu Leu Ser Asp
                    290                  295                  300
        Ile Leu Arg Val Asn Thr Glu Ile Thr Lys Ala Pro Leu Ser Ala Ser
        305                  310                  315                  320
        Met Ile Lys Arg Tyr Asp Glu His His Gln Asp Leu Thr Leu Leu Lys
                    325                  330                  335
        Ala Leu Val Arg Gln Gln Leu Pro Glu Lys Tyr Lys Glu Ile Phe Phe
                    340                  345                  350
        Asp Gln Ser Lys Asn Gly Tyr Ala Gly Tyr Ile Asp Gly Gly Ala Ser
                    355                  360                  365
        Gln Glu Glu Phe Tyr Lys Phe Ile Lys Pro Ile Leu Glu Lys Met Asp
                    370                  375                  380
        Gly Thr Glu Glu Leu Leu Val Lys Leu Asn Arg Glu Asp Leu Leu Arg
        385                  390                  395                  400
        Lys Gln Arg Thr Phe Asp Asn Gly Ser Ile Pro His Gln Ile His Leu
                    405                  410                  415
        Gly Glu Leu His Ala Ile Leu Arg Arg Gln Glu Asp Phe Tyr Pro Phe
                    420                  425                  430
        Leu Lys Asp Asn Arg Glu Lys Ile Glu Lys Ile Leu Thr Phe Arg Ile
                    435                  440                  445
        Pro Tyr Tyr Val Gly Pro Leu Ala Arg Gly Asn Ser Arg Phe Ala Trp
                    450                  455                  460
        Met Thr Arg Lys Ser Glu Glu Thr Ile Thr Pro Trp Asn Phe Glu Glu
        465                  470                  475                  480
        Val Val Asp Lys Gly Ala Ser Ala Gln Ser Phe Ile Glu Arg Met Thr
                    485                  490                  495
        Asn Phe Asp Lys Asn Leu Pro Asn Glu Lys Val Leu Pro Lys His Ser
                    500                  505                  510
        Leu Leu Tyr Glu Tyr Phe Thr Val Tyr Asn Glu Leu Thr Lys Val Lys
                    515                  520                  525
        Tyr Val Thr Glu Gly Met Arg Lys Pro Ala Phe Leu Ser Gly Glu Gln
                    530                  535                  540
        Lys Lys Ala Ile Val Asp Leu Leu Phe Lys Thr Asn Arg Lys Val Thr
        545                  550                  555                  560
        Val Lys Gln Leu Lys Glu Asp Tyr Phe Lys Lys Ile Glu Cys Phe Asp
                    565                  570                  575
        Ser Val Glu Ile Ser Gly Val Glu Asp Arg Phe Asn Ala Ser Leu Gly
                    580                  585                  590
        Thr Tyr His Asp Leu Leu Lys Ile Ile Lys Asp Lys Asp Phe Leu Asp
                    595                  600                  605
        Asn Glu Glu Asn Glu Asp Ile Leu Glu Asp Ile Val Leu Thr Leu Thr
                    610                  615                  620
        Leu Phe Glu Asp Arg Glu Met Ile Glu Glu Arg Leu Lys Thr Tyr Ala
        625                  630                  635                  640
        His Leu Phe Asp Asp Lys Val Met Lys Gln Leu Lys Arg Arg Arg Tyr
                    645                  650                  655
```

18

```
Thr Gly Trp Gly Arg Leu Ser Arg Lys Leu Ile Asn Gly Ile Arg Asp
        660             665             670
Lys Gln Ser Gly Lys Thr Ile Leu Asp Phe Leu Lys Ser Asp Gly Phe
        675             680             685
Ala Asn Arg Asn Phe Met Gln Leu Ile His Asp Asp Ser Leu Thr Phe
        690             695             700
Lys Glu Asp Ile Gln Lys Ala Gln Val Ser Gly Gln Gly Asp Ser Leu
705             710             715             720
His Glu His Ile Ala Asn Leu Ala Gly Ser Pro Ala Ile Lys Lys Gly
        725             730             735
Ile Leu Gln Thr Val Lys Val Val Asp Glu Leu Val Lys Val Met Gly
        740             745             750
Arg His Lys Pro Glu Asn Ile Val Ile Glu Met Ala Arg Glu Asn Gln
        755             760             765
Thr Thr Gln Lys Gly Gln Lys Asn Ser Arg Glu Arg Met Lys Arg Ile
        770             775             780
Glu Glu Gly Ile Lys Glu Leu Gly Ser Gln Ile Leu Lys Glu His Pro
785             790             795             800
Val Glu Asn Thr Gln Leu Gln Asn Glu Lys Leu Tyr Leu Tyr Tyr Leu
        805             810             815
Gln Asn Gly Arg Asp Met Tyr Val Asp Gln Glu Leu Asp Ile Asn Arg
        820             825             830
Leu Ser Asp Tyr Asp Val Asp Ala Ile Val Pro Gln Ser Phe Leu Lys
        835             840             845
Asp Asp Ser Ile Asp Asn Lys Val Leu Thr Arg Ser Asp Lys Asn Arg
850             855             860
Gly Lys Ser Asp Asn Val Pro Ser Glu Glu Val Val Lys Lys Met Lys
865             870             875             880
Asn Tyr Trp Arg Gln Leu Leu Asn Ala Lys Leu Ile Thr Gln Arg Lys
        885             890             895
Phe Asp Asn Leu Thr Lys Ala Glu Arg Gly Gly Leu Ser Glu Leu Asp
        900             905             910
Lys Ala Gly Phe Ile Lys Arg Gln Leu Val Glu Thr Arg Gln Ile Thr
        915             920             925
Lys His Val Ala Gln Ile Leu Asp Ser Arg Met Asn Thr Lys Tyr Asp
        930             935             940
Glu Asn Asp Lys Leu Ile Arg Glu Val Lys Val Ile Thr Leu Lys Ser
945             950             955             960
Lys Leu Val Ser Asp Phe Arg Lys Asp Phe Gln Phe Tyr Lys Val Arg
        965             970             975
Glu Ile Asn Asn Tyr His His Ala His Asp Ala Tyr Leu Asn Ala Val
        980             985             990
Val Gly Thr Ala Leu Ile Lys Lys  Tyr Pro Lys Leu Glu  Ser Glu Phe
        995             1000            1005
Val Tyr  Gly Asp Tyr Lys Val  Tyr Asp Val Arg Lys  Met Ile Ala
        1010            1015            1020
Lys Ser  Glu Gln Glu Ile Gly  Lys Ala Thr Ala Lys  Tyr Phe Phe
        1025            1030            1035
Tyr Ser  Asn Ile Met Asn Phe  Phe Lys Thr Glu Ile  Thr Leu Ala
        1040            1045            1050
Asn Gly  Glu Ile Arg Lys Arg  Pro Leu Ile Glu Thr  Asn Gly Glu
        1055            1060            1065
Thr Gly  Glu Ile Val Trp Asp  Lys Gly Arg Asp Phe  Ala Thr Val
        1070            1075            1080
Arg Lys  Val Leu Ser Met Pro  Gln Val Asn Ile Val  Lys Lys Thr
        1085            1090            1095
Glu Val  Gln Thr Gly Gly Phe  Ser Lys Glu Ser Ile  Leu Pro Lys
        1100            1105            1110
Arg Asn  Ser Asp Lys Leu Ile  Ala Arg Lys Lys Asp  Trp Asp Pro
        1115            1120            1125
Lys Lys  Tyr Gly Gly Phe Asp  Ser Pro Thr Val Ala  Tyr Ser Val
```

```
           1130                      1135                       1140
     Leu Val Val Ala Lys Val Glu Lys Gly Lys Ser Lys Lys Leu Lys
           1145                      1150                       1155
     Ser Val Lys Glu Leu Leu Gly Ile Thr Ile Met Glu Arg Ser Ser
           1160                      1165                       1170
     Phe Glu Lys Asn Pro Ile Asp Phe Leu Glu Ala Lys Gly Tyr Lys
           1175                      1180                       1185
     Glu Val Lys Lys Asp Leu Ile Ile Lys Leu Pro Lys Tyr Ser Leu
           1190                      1195                       1200
     Phe Glu Leu Glu Asn Gly Arg Lys Arg Met Leu Ala Ser Ala Gly
           1205                      1210                       1215
     Glu Leu Gln Lys Gly Asn Glu Leu Ala Leu Pro Ser Lys Tyr Val
           1220                      1225                       1230
     Asn Phe Leu Tyr Leu Ala Ser His Tyr Glu Lys Leu Lys Gly Ser
           1235                      1240                       1245
     Pro Glu Asp Asn Glu Gln Lys Gln Leu Phe Val Glu Gln His Lys
           1250                      1255                       1260
     His Tyr Leu Asp Glu Ile Ile Glu Gln Ile Ser Glu Phe Ser Lys
           1265                      1270                       1275
     Arg Val Ile Leu Ala Asp Ala Asn Leu Asp Lys Val Leu Ser Ala
           1280                      1285                       1290
     Tyr Asn Lys His Arg Asp Lys Pro Ile Arg Glu Gln Ala Glu Asn
           1295                      1300                       1305
     Ile Ile His Leu Phe Thr Leu Thr Asn Leu Gly Ala Pro Ala Ala
           1310                      1315                       1320
     Phe Lys Tyr Phe Asp Thr Thr Ile Asp Arg Lys Arg Tyr Thr Ser
           1325                      1330                       1335
     Thr Lys Glu Val Leu Asp Ala Thr Leu Ile His Gln Ser Ile Thr
           1340                      1345                       1350
     Gly Leu Tyr Glu Thr Arg Ile Asp Leu Ser Gln Leu Gly Gly Asp
           1355                      1360                       1365
```

In some examples, the technology comprises use of a nucleotide sequence that is approximately 50%, 55%, 60%, 65%, 70%, 75%, 80%, 85%, 90%, 95%, 99% or 100% identical to a nucleotide sequence that encodes a protein described by SEQ ID NO: 1.

In some examples, the dCas9 used herein is at least about 50% identical to the sequence of the catalytically inactive *S. pyogenes* Cas9, i.e., at least 50%, 55%, 60%, 65%, 70%, 75%, 80%, 85%, 90%, 95%, 99% or 100% identical to SEQ ID NO:1, wherein the mutations at D10 and H840, e.g., D10A/D10N and H840A/H840N/H840Y are maintained.

In some examples, any differences from SEQ ID NO:1 are in non-conserved regions, as identified by sequence alignment of sequences set forth in Chylinski et al., RNA Biology 10:5, 1-12; 2013 (e.g., in supplementary FIG. 1 and supplementary table 1 thereof); Esvelt et al., Nat Methods. 2013 November; 10(11):1116-21 and Fonfara et al., Nucl. Acids Res. (2014) 42 (4): 2577-2590. [Epub ahead of print 2013 Nov. 22] doi:10.1093/nar/gkt1074, and wherein the mutations at D10 and H840, e.g., D10A/D10N and H840A/H840N/H840Y are maintained.

To determine the percent identity of two sequences, the sequences are aligned for optimal comparison purposes (gaps are introduced in one or both of a first and a second amino acid or nucleic acid sequence as required for optimal alignment, and non-homologous sequences can be disregarded for comparison purposes). The length of a reference sequence aligned for comparison purposes is at least 50% (in some examples, about 50%, 55%, 60%, 65%, 70%, 75%, 85%, 90%, 95%, or 100% of the length of the reference sequence) is aligned. The nucleotides or residues at corresponding positions are then compared. When a position in the first sequence is occupied by the same nucleotide or residue as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. For purposes of the present application, the percent identity between two amino acid sequences is determined using the Needleman and Wunsch ((1970) J. Mol. Biol. 48:444-453) algorithm which has been incorporated into the GAP program in the GCG software package, using a Blosum 62 scoring matrix with a gap penalty of 12, a gap extend penalty of 4, and a frameshift gap penalty of 5.

Accordingly, provided herein is a dCas9/gRNA-based approach for detecting double-stranded nucleic acids (e.g., double-stranded genomic DNA) using SiMREPS probes. Examples of the technology comprise capturing unlabeled genomic DNA targets on a glass or fused silica surface using a gRNA-loaded, enzymatically dead dCas9 enzyme or enzymes that bind one or more segments of the target with high specificity and stability (e.g., forming approximately 20 base pairs at the site complementary to the gRNA; Fig. 3). Surface capture of nucleic acid targets by biotinylated dCas9 is followed by observing repeated, transient binding of the SiMREPS probe to a second segment of the target that has been made accessible by dCas9/gRNA-mediated melting.

Thus, in some examples a biotinylated dCas9 comprising a guide RNA (gRNA) comprising an appropriate sequence captures a target nucleic acid. In addition, dCas9, with the help of the gRNA, melts the DNA, which produces a complementary non-template DNA strand that is single-stranded and accessible to the query probe. In some examples, the technology comprises use of a second non-biotinylated dCas9 comprising a second gRNA that binds to the target nucleic acid (Fig. 4). The second dCas9/gRNA binds to the target nucleic acid at a distance from the first dCas9/gRNA that is approximately the size of the query probe, e.g., 5 to 30 nucleotides. That is, the biotinylated dCas9/gRNA (capture dCas9/gRNA) and second dCas9/gRNA bind to regions adjacent to the region of the target nucleic acid to which the query probe binds. The two dCas9/gRNA complexes melt the region of nucleic acid between them to provide a single-stranded query region accessible for query probe binding. Accordingly, the spacing between the two dCas9/gRNA complexes has is appropriate for the binding of a query probe between them.

## Methods

In some examples, the technology provides a method for detecting a double-stranded nucleic acid. For example, in some examples a nucleic acid (e.g., a genomic DNA) target DNA is briefly pre-treated with dCas9/gRNA (e.g., at or near ambient ("room") temperature). Next, the guide RNA (gRNA) of the dCas9/gRNA complex hybridizes to the target nucleic acid (e.g., a genomic DNA) at a region adjacent to a query region (e.g., a region of the target nucleic acid that is complementary to a query probe). The dCas9/gRNA melts a specific DNA sequence (e.g., the query region) in the target nucleic acid. Methods comprise capturing the dCas9 (e.g., a biotinylated dCas9) onto a slide surface (e.g., by biotin-avidin interaction). Following capture and immobilization of the dCas9/gRNA-target nucleic acid to the surface, SiMREPS is used to detect binding of a query probe to the query region in the target nucleic acid, which is adjacent to the site of target nucleic acid hybridized to the gRNA. See, e.g., Fig. 3.

In some examples, two dCas9/gRNA complexes are used to make the query region of a target nucleic acid accessible to a query probe. In some examples, hybridizing two dCas9/gRNA complexes to flank the query region improves accessibility of the target nucleic acid (e.g., the query region) to the query probe for SiMREPS-based detection. One dCas9 is biotinylated for capture onto the surface; the other (e.g., second) dCas9 is optionally biotinylated (in preferred embodiments, the second dCas9 is not biotinylated). See, e.g., Fig. 4. The space between the regions bound by the two dCas9/gRNA complexes bound to the target nucleic acid provides appropriate space for binding of the query probe to the query region of the target nucleic acid.

In some examples, the detectable (e.g., fluorescent) query probe produces a fluorescence emission signal when it is close to the surface of the solid support (e.g., within about 100 nm of the surface of the solid support). When unbound, query probes quickly diffuse and thus are not individually detected; accordingly, when in the unbound state, the query probes produce a low level of diffuse background fluorescence. Consequently, in some examples detection of bound query probes comprises use of total internal reflection fluorescence microscopy (TIRF), HiLo microscopy (see, e.g., US20090084980, EP2300983 B1, WO2014018584 A1, WO2014018584 A1), confocal scanning microscopy, or other technologies comprising illumination schemes that illuminate (e.g., excite) only those query probe molecules near or on the surface of the solid support. Thus, in some examples, only query probes that are bound to an immobilized target near or on the surface produce a point-like emission signal (e.g., a "spot") that can be confirmed as originating from a single molecule.

In general terms, the observation comprises monitoring fluorescence emission at a number of discrete locations on the solid support where the target nucleic acids are immobilized (e.g., by being specifically bound to the dCas9/gRNA attached to the surface), e.g., at a number of fluorescent "spots" that blink, e.g., that can be in "on" and "off" states. The presence of fluorescence emission (spot is "on") and absence of fluorescence emission (spot is "off") at each discrete location (e.g., at each "spot" on the solid support) are recorded. Each spot "blinks" - e.g., a spot alternates between "on" and "off" states, respectively, as a query probe binds to the immobilized target nucleic acid at that spot and as the query probe dissociates from the immobilized target nucleic acid at that spot.

The data collected provide for the determination of the number of times a query probe binds to each immobilized target (e.g., the number of times each spot blinks "on") and a measurement of the amount of time a query probe remains bound (e.g., the length of time a spot remains "on" before turning "off").

In some examples, the query probe comprises a fluorescent label having an emission wavelength. Detection of fluorescence emission at the emission wavelength of the fluorescent label indicates that the query probe is bound to an immobilized target nucleic acid. Binding of the query probe to the target nucleic acid is a "binding event". In some examples of the technology, a binding event has a fluorescence emission having a measured intensity greater than a defined threshold. For example, a binding event may have a fluorescence intensity that is above the background fluorescence intensity (e.g., the fluorescence intensity observed in the absence of a target nucleic acid). In some examples, a binding event has a fluorescence intensity that is at least 1, 2, 3, 4 or more standard deviations above the background fluorescence intensity (e.g., the fluorescence intensity observed in the absence of a target nucleic acid). In some examples, a binding event has a fluorescence intensity that is at least 2 standard deviations above the background fluorescence intensity (e.g., the fluorescence intensity observed in the absence of a target nucleic acid). In some examples, a binding event has a fluorescence intensity that is at least 1.5, 2, 3, 4, or 5 times the background fluorescence intensity (e.g., the mean fluorescence intensity observed in the absence of a target nucleic acid).

Accordingly, in some examples detecting fluorescence at the emission wavelength of the fluorescent probe that has an intensity above the defined threshold (e.g., at least 2 standard deviations greater than background intensity) indicates that a binding event has occurred (e.g., at a discrete location on the solid support where a target nucleic acid is immobilized). Also, in some examples detecting fluorescence at the emission wavelength of the fluorescent probe that has an intensity above the defined threshold (e.g., at least 2 standard deviations greater than background intensity) indicates that a binding event has started. Accordingly, in some examples detecting an absence of fluorescence at the emission wavelength of the fluorescent probe that has an intensity above the defined threshold (e.g., at least 2 standard deviations greater than background intensity) indicates that a binding event has ended (e.g., the query probe has dissociated from the target nucleic acid). The length of time between when the binding event started and when the binding event ended (e.g., the length of time that fluorescence at the emission wavelength of the fluorescent probe having an intensity above the defined threshold (e.g., at least 2 standard deviations greater than background intensity) is detected) is the dwell time of the binding event. A "transition" refers to the binding and dissociation of a query probe to the target nucleic acid (e.g., an on/off event).

Methods according to the technology comprise counting the number of query probe binding events that occur at each discrete location on the solid support during a defined time interval that is the "acquisition time" (e.g., a time interval that is tens to hundreds to thousands of seconds, e.g., 5, 10, 15, 20, 25, 30, 35, 40, 45, 50, 55, or 60 seconds; e.g., 5, 10, 15, 20, 25, 30, 35, 40, 45, 50, 55, or 0 minutes; e.g., 1, 1.5, 2, 2.5, or 3 hours). In some examples, the acquisition time is approximately 1 to 10 seconds to 1 to 10 minutes (e.g., approximately 1 to 100 seconds, e.g., 1, 2, 3, 4, 5, 6, 7, 8, 9, 10, 11, 12, 13, 14, 15, 16, 17, 18, 19, 20, 30, 40, 50, 60, 70, 80, 90, or 100 seconds, e.g., 1 to 100 minutes, e.g., 1, 2, 3, 4, 5, 6, 7, 8, 9, 10, 11, 12, 13, 14, 15, 16, 17, 18, 19, 20, 30, 40, 50, 60, 70, 80, 90, or 100 minutes).

Further, the length of time the query probe remains bound to the target nucleic acid during a binding event is the "dwell time" of the binding event. The number of binding events detected during the acquisition time and/or the lengths of the dwell times recorded for the binding events is/are characteristic of a query probe binding to a target nucleic acid and thus provide an indication that the target nucleic acid is immobilized at said discrete location and thus that the target nucleic acid is present in the sample.

Binding of the query probe to the immobilized target nucleic acid and/or and dissociation of the query probe from the immobilized target nucleic acid is/are monitored (e.g., using a light source to excite the fluorescent probe and detecting fluorescence emission from a bound query probe, e.g., using a fluorescence microscope) and/or recorded during a defined time interval (e.g., during the acquisition time). The number of times the query probe binds to the nucleic acid during the acquisition time and/or the length of time the query probe remains bound to the nucleic acid during each binding event and the length of time the query probe remains unbound to the nucleic acid between each binding event (e.g., the "dwell times" in the bound and unbound states, respectively) are determined, e.g., by the use of a computer and software (e.g., to analyze the data using a hidden Markov model and Poisson statistics).

In some examples, control samples are measured (e.g., in absence of target). Fluorescence detected in a control sample is "background fluorescence" or "background (fluorescence) intensity" or "baseline".

In some examples, data comprising measurements of fluorescence intensity at the emission wavelength of the query probe are recorded as a function of time. In some examples, the number of binding events and the dwell times of binding events (e.g. for each immobilized nucleic acid) are determined from the data (e.g., by determining the number of times and the lengths of time the fluorescence intensity is above a threshold background fluorescence intensity). In some examples, transitions (e.g., binding and dissociation of a query probe) are counted for each discrete location on the solid support where a target nucleic acid is immobilized. In some examples, a threshold number of transitions is used to discriminate the presence of a target nucleic acid at a discrete location on the solid support from background signal, non-target nucleic acid, and/or spurious binding of the query probe. In some examples, a number of transitions greater than 10 recorded during the acquisition time indicates the presence of a target nucleic acid at the discrete location on the solid support.

In some examples, a distribution of the number of transitions for each immobilized target is determined - e.g., the number of transitions is counted for each immobilized nucleic acid target observed. In some examples a histogram is produced. In some examples, characteristic parameters of the distribution are determined, e.g., the mean, median, peak, shape, etc. of the distribution are determined. In some examples, data and/or parameters (e.g., fluorescence data (e.g., fluorescence data in the time domain), kinetic data, characteristic parameters of the distribution, etc.) are analyzed by algorithms that recognize patterns and regularities in data, e.g., using artificial intelligence, pattern recognition, machine learning, statistical inference, neural nets, etc. In some examples, the analysis comprises use of a frequentist analysis and in some examples the analysis comprises use of a bayesian analysis. In some examples, pattern recognition systems are trained using known "training" data (e.g., using supervised learning) and in some examples algorithms are used to discover previously unknown patterns (e.g., unsupervised learning). See, e.g., Duda, et al. (2001) Pattern classification (2nd edition), Wiley, New York; Bishop (2006) Pattern Recognition and Machine Learning, Springer.

Pattern recognition (e.g., using training sets, supervised learning, unsupervised learning, and analysis of unknown samples) associates identified patterns with nucleic acids such that particular patterns provide a "fingerprint" of particular nucleic acids that find use in detection, quantification, and identification of nucleic acids.

In some examples, the distribution produced from a target nucleic acid is significantly different than a distribution produced from a non-target nucleic acid or the distribution produced in the absence of a target nucleic acid. In some examples, a mean number of transitions is determined for the plurality of immobilized target nucleic acids. In some examples, the mean number of transitions observed for a sample comprising a target nucleic acid is approximately linearly related as a function of time and has a positive slope (e.g., the mean number of transitions increases approximately linearly as a function of time).

In some examples, the data are treated using statistics (e.g., Poisson statistics) to determine the probability of a transition occurring as a function of time at each discrete location on the solid support. In some particular examples, a relatively constant probability of a transition event occurring as a function of time at a discrete location on the solid support indicates the presence of a target nucleic acid at said discrete location on the solid support. In some examples, a correlation coefficient relating event number and elapsed time is calculated from the probability of a transition event occurring as a function of time at a discrete location on the solid support. In some examples, a correlation coefficient relating event number and elapsed time greater than 0.95 when calculated from the probability of a transition event occurring as a function of time at a discrete location on the solid support indicates the presence of a target nucleic acid at said discrete location on the solid support.

In some examples, dwell times of bound query probe ($\tau_{on}$) and unbound query probe ($\tau_{off}$) are used to identify the presence of a target nucleic acid in a sample and/or to distinguish a sample comprising a target nucleic acid from a sample comprising a non-target nucleic acid and/or not comprising the target nucleic acid. For example, the $\tau_{on}$ for a target nucleic acid is greater than the $\tau_{on}$ for a non-target nucleic acid; and, the $\tau_{off}$ for a target nucleic acid is smaller than the $\tau_{off}$ for a non-target nucleic acid. In some examples, measuring $\tau_{on}$ and $\tau_{off}$ for a negative control and for a sample indicates the presence or absence of the target nucleic acid in the sample. In some examples, a plurality of $\tau_{on}$ and $\tau_{off}$ values is determined for each of a plurality of spots imaged on a solid support, e.g., for a control (e.g., positive and/or negative control) and a sample suspected of comprising a target nucleic acid. In some examples, a mean $\tau_{on}$ and/or $\tau_{off}$ is determined for each of a plurality of spots imaged on a solid support, e.g., for a control (e.g., positive and/or negative control) and a sample suspected of comprising a target nucleic acid. In some examples, a plot of $\tau_{on}$ versus $\tau_{off}$ (e.g., mean $\tau_{on}$ and $\tau_{off}$, time-averaged $\tau_{on}$ and $\tau_{off}$, etc.) for all imaged spots indicates the presence or absence of the target nucleic acid in the sample.

**Applications**

The technology finds use in the detection of nucleic acids, e.g., single-stranded and double-stranded nucleic acids. Accordingly, the technology provides for the detection of various forms of DNA (e.g., DNA comprising modified bases, e.g., methylated DNA, unmethylated DNA) and RNA (e.g., lncRNA, miRNA, etc.), e.g., to provide multi-nucleic acid detection on a common platform. Accordingly, the technology finds use in exemplary applications such as, e.g., detecting one or more of microRNAs, RNAs, mutant DNA alleles, wild-type DNA alleles, and locus-specific methylated DNAs, all on the SiMREPS platform. Detection of these types of nucleic acids occurs, in some examples, in the same sample.

For example, the technology finds use in detecting methylated DNA. Methylated DNA is a marker for many states of health and disease, including, for example, identifying patients at higher risk of colorectal cancer based on presence of specific methylated loci. Methylated DNA also provides the basis of a diagnostic test for the early detection of colorectal cancer as well as pre-cancerous adenomas and dysplastic lesions. Detection of methylated DNA at specific loci is currently performed by sodium bisulfite treatment of DNA, which deaminates unmethylated cytosines to produce uracil in the DNA, followed by PCR using distinct primer sets that selectively amplify methylated DNA fragments (e.g., 5-mC or 5-hmC, which are protected from conversion by Na bisulfite) or the unmethylated fragments (where primers are designed to bind to the anticipated converted sequence containing uracils in place of cytosines). Another approach is to perform next generation sequencing of bisulfite treated and/or untreated DNA to infer methylated bases.

Although bisulfite conversion followed by PCR or next generation sequencing are commonly used approaches, they suffer from significant limitations. Surprising, some of these limitations of bisulfite based technologies provide advantages for detection of methylated DNA using the SiMREPS technology provided herein. First, the bisulfite treatment not only deaminates cytosines but also randomly fragments the DNA into shorter fragments, creating challenges for designing PCR primers, especially as the sequence length gets very short. In addition, following bisulfite conversion, the regions of DNA that have undergone deamination at cytosines are no longer complementary and therefore not double-stranded, which makes PCR somewhat more challenging as only one strand can be amplified with any given PCR primer set. A SiMREPS approach, on the other hand, fundamentally benefits from the conversion of double-stranded nucleic acids to single-stranded nucleic acids, as well as from fragmentation of nucleic acids to short fragments.

In some examples, the presence or absence of nucleic acid modifications are detected (e.g., modified bases, nucleotide analogs, etc.). For example, epigenetic modifications of nucleic acids that influence gene expression can be detected. In some examples, methylation of DNA is detected. In some examples, the technology finds use in detecting (e.g., identifying the presence or absence of) nucleotide analogs, nucleotide bases - or nucleosides and/or nucleotides comprising bases - other than adenine, thymine, guanosine, cytosine, and uracil. For example, the technology may find use in detecting, identifying, and/or quantifying a nucleotide, nucleoside, and/or abase including but not limited to, e.g., 5-methylcytosine (5mC), 5-hydroxymethylcytosine (5hmC), 5-formylcytosine (5fC), and 5-carboxycytosine (5caC), N(6)-methyladenosine (m(6)A), pseudouridine (Ψ), dihydrouridine (D), inosine (I), 7-methylguanosine (m7G), hypoxanthine, xanthine, 2,6-diaminopurine, and 6,8-diaminopurine.

In an example, the technology comprises detecting methylated DNA by SiMREPS comprises analyzing samples that are treated with bisulfite and samples that have not been treated with bisulfite. Examples provide using SiMREPS query probes that distinguish sequences expected from conversion of unmethylated cytosines to uracil from sequences expected if cytosine(s) at a given locus are not converted to uracil (due to methylation). In some examples, both query probes are provided in the sample chamber at the same time and each probe comprises a different fluorophore. In some examples, bisulfite reagent is provided in real-time while imaging a SiMREPS experiment with both query probes present (e.g., one probe that binds to the methylated sequence and a second probe that binds to the uracil-converted sequence, each with a separate fluorophore), where DNA fragments blinking in one color would shift to blinking in the other color based on conversion. Such an assay provides greater accuracy and precision in the measurements than comparing a bisulfite-treated aliquot of sample to an untreated aliquot, which is required for current PCR or next generation sequencing-based approaches. In some examples, the technology is a multiplexed analysis using microfluidics and/or multi-spectral imaging, for example. In addition to bisulfate modification, the technology comprises use of other reagents that convert additional chemical markers on DNA or RNA into modifications that are easily detected by SiMREPS.

In some examples, the technology finds use in detecting a combination of mutant DNA, methylated DNA, and microRNA biomarkers on the same platform. For example, such a technology finds use in the detection of colorectal cancer and advanced adenoma, e.g., by analyzing a stool sample. The technology provides for the detection of mutant DNA (e.g., detecting 1 mutant molecule in a background of 1,000,000 wild-type molecules; e.g., detecting KRAS mutant DNA in a background of wild-type DNA). Moreover, the technology also provides a technology for measuring all three types of markers (methylated DNA, miRNA, and/or mutant DNA). The technology finds use in analyzing nucleic acids in a buffer solution; the technology finds use in analyzing nucleic acids in a matrix extracted from stool. An average stool weighs 200 g and comprises approximately 10 million diploid-genome equivalents of human DNA. A sensitivity of 1:1,000,000 provides detection of as few as 10 mutant molecules in DNA extracted from a typical whole stool. This is 10,000-fold more sensitive than current clinically-used KRAS assays and 100-fold more sensitive than best-performing research-grade methods. See, e.g., Domagala et al. (2012) "KRAS mutation testing in colorectal cancer as an example of the pathologist's role in personalized targeted therapy: a practical approach" Pol J Pathol 63(3): 145-64; Gerecke et al. (2013) "Ultrasensitive detection of unknown colon cancer-initiating mutations using the example of the Adenomatous polyposis coli gene" Cancer Prev Res (Phila). 6(9): 898-907. This dramatic advance in performance is provided by the arbitrary specificity for allele discrimination that is inherent to the kinetic fingerprinting of the SiMREPS technology.

## Detecting biomarkers for cancer

Colonoscopy is the dominant screening approach for colorectal cancer (CRC) in the U.S., despite its invasiveness, high cost, low patient compliance, and risk of complications. New diagnostics, such as stool-based colorectal cancer screening, are limited by low sensitivity for detecting advanced adenomas (AA), removal of which prevents CRC. The currently best-performing stool-based test analyzes stool DNA (mutant DNA and methylation) and occult blood, but is technically complex, expensive ($500/test), and challenged by limited ability to detect rare mutant DNA alleles in a high background of wild-type DNA.

The detection technology described herein provides a new technology for low-cost, rapid measurement of rare mutant DNA alleles in stool with exquisite analytic specificity, with concurrent measurement of occult blood (e.g., using a microRNA marker of occult blood) and methylated DNA on a single platform. The technology provides increased sensitivity for detecting advanced adenoma at a >10-fold lower cost than the current state-of-the-art.

The technology provides quantification of rare mutant DNA alleles with orders of magnitude higher specificity than current methods, leading to significantly sensitized AA detection. Adenoma-defining mutations such as in the APC gene can be detected.

The technology provides for the measurement of multiple stool biomarker types, all on a single platform. In some examples, in addition to mutant DNA, markers detected on the platform include microRNA (see, e.g., US 2016/0046988 A1), a stool occult blood marker (e.g., a microRNA marker of stool occult blood), and methylated DNA.

**Detection of RNA**

In some examples, the nucleic acid to be detected, characterized, quantified, and/or identified (e.g., the target nucleic acid) is a RNA (e.g., a lncRNA, e.g., a non-protein coding RNA longer than approximately 200 nucleotides). Examples provide that the secondary structure of a nucleic acid (e.g.,a RNA, e.g., a lncRNA) is melted to provide access to query regions by query probes for SiMREPS detectino. For example, the technology may comprise use of a dCas9/gRNA that recognizes and melts a secondary structured target in an RNA using an auxiliary PAM oligonucleotide (e.g., a PAMmer). In some examples the technology comprises use of two dCas9/gRNA complexes that recognize and melt a secondary structured target in an RNA using an auxiliary PAM oligonucleotide (e.g., a PAMmer).

In some examples, the dCas9 binds to single-stranded RNA targets matching the gRNA sequence when the PAM is presented in trans as a separate DNA oligonucleotide (a "PAM-presenting oligonucleotide" or "PAMmer"). Accordingly, in some examples PAMmers provide for the site-specific binding of dCas9/gRNA to single-stranded RNA targets (e.g., lncRNA). Furthermore, the technology provides for the use of PAMmers to direct dCas9 to bind to specific RNA targets and melt secondary structure to make query regions available for the binding of query probes in a SiMREPs assay. See, e.g., O'Connell et al. 2014 "Programmable RNA recognition and cleavage by CRISPR/Cas9" Nature 516: 263-6. Thus, examples provide compositions comprising a dCas9, a gRNA (e.g., a dCas9/gRNA complex), and a PAMmer.

**Detection of microRNA**

In some examples, the nucleic acid to be detected, characterized, quantified, and/or identified (e.g., the target nucleic acid) is a microRNA. microRNAs (miRNA or $\mu$RNA) are single-stranded RNA molecules of approximately 21 to 23 nucleotides in length that regulate gene expression. miRNAs are encoded by genes from whose DNA they are transcribed, but miRNAs are not translated into protein (see, e.g., Carrington et al, 2003). The genes encoding miRNAs are much longer than the processed mature miRNA molecule. See, e.g., US 2016/0046988 A1.

**Detection of genomic aberrations**

In some examples, the SiMREPS technology provides for identifying and/or counting genomic aberrations (e.g., other than simple point mutations) in a DNA sample based on detecting unpaired regions after comparative genome hybridization. In some examples, SiMREPS finds use to determine the overall degree of genomic instability in a sample, e.g., as evidenced by presence of deletions and insertions compared to a reference, normal DNA sample. Exemplary examples comprise providing a normal DNA (for example, wild-type DNA from normal blood cells of a patient with a solid tumor like lung cancer) and mix it with matched tumor DNA (or even circulating cell-free DNA), with fragmentation of the DNA so that it is present as fragments and is immobilized onto a surface, potentially through end modification (e.g., biotinylation) or other approaches. Most of the DNA will form hybridized DNA segments, but areas of deletion or insertion are present as duplexes where one strand bulges out. In some examples, the normal DNA and the tumor DNA are provided in ratios appropriate for efficient detection. Examples provide a SiMREPS-based affinity reagent that is used to detect these unmatched regions, and counting them provides a measure of genomic instability, which finds use, e.g., in some examples as a biomarker of cancer risk. In some examples, the technology finds use in pre-natal screening for chromosomal abnormalities. In some examples, the affinity reagent is a single-stranded DNA binding proteins, a Holliday junction recombinases modified not to cleave nucleic acid, e.g., for identification of balanced chromosomal translocations, etc.

In some examples, the technology finds use in the detection of microsatellite repeat aberrations in patients with microsatellite-unstable colorectal cancer, for example. Accordingly, examples comprise providing a panel of SiMREPS probes corresponding to different microsatellite loci that show differential kinetic binding properties depending on whether the microsatellite repeats have expanded or not in a sample. In some examples, the technology comprises a comparative DNA hybridization approach in which hybridization of an expanded microsatellite repeat sequence to one without expansion generates an unpaired segment that is detected using a SiMREPS-based approach, using a DNA probe or other effective affinity SiMREPS reader reagent.

## Bifunctional affinity reagents

In some examples, the technology comprises detection of proteins and other analytes with SiMREPS by incorporation of a bifunctional affinity reagent that binds to the target analyte and comprises a nucleic acid that can be counted using a SiMREPS reader probe. As an example, the use of an antibody linked to a short DNA oligonucleotide may be comprised, such that if the target protein analyte were immobilized onto the surface of a slide (for example by simple drying onto the surface, or other nonspecific or specific capture methods), the binding of the antibody to the target protein analyte is measured using a SiMREPS-based reading of the conjugated DNA. This would allow multiple antibodies to be multiplexed and distinguished by different DNA sequence "barcodes" linked to the various antibodies. Examples provide that the samples are a large variety of types including even cells or cell lysates. Affinity reagents include DNA or RNA binding proteins, aptamers, antibodies and antibody fragments, linked to a DNA barcode.

## Non-nucleic acid SiMREPS probes

Examples provide probes that are not nucleic acids. For example, an antibody or other affinity reagent that have a binding interaction with a target analyte that has a stability amenable for SiMREPS may be provided, e.g., a transient associated that provides a "blinking" signal and, in some examples, a kinetic binding fingerprint. In some examples, the non-nucleic acid query probe is engineered to weaken its binding relative to the non-engineered version, e.g., to provide a binding and/or association that is less thermodynamically stable. In addition to antibodies, examples comprise target analytes and query probes that are proteins, e.g., where one binding partner is the affinity reagent and the other would is the target analyte being measured. Examples comprise the use of aptamers binding any ligand, lectins binding glycosylated proteins, proteins or other molecules binding lipids, etc. The technology comprises the use of any binding pair with transient binding behavior suitable for detection on the SiMREPS platform, e.g., that produces a kinetic fingerprint.

## Intramolecular SiMREPS probing

In some examples, the technology provides a capture probe and a query probe that are linked to provide an intramolecular probing mechanism (see, e.g., Fig. 5 a and b). The probe is asymmetric, so that when the target nucleic acid binds (e.g., with thermodynamic stability, e.g., irreversibly) to the capture sequence, the target nucleic acid undergoes transient binding with the query probe. The transient binding and dissociation of the query probe yields a time-dependent change in donor fluorophore intensity or FRET whose kinetics are sensitive to the sequence of the target nucleic acid. In some examples, an address strand binds the Query/Capture complex to the surface, to provide rigidity that exerts control over the transient binding kinetics, and to provide a means to immobilize many different Query/Capture sequences to different regions of the imaging surface (e.g., as in a DNA microarray).

In some examples, intramolecular SiMREPS probing provides faster acquisition. In particular, binding of the query probe is rapid because the binding is an intramolecular hybridization reaction after the target nucleic acid binds to the capture probe. Furthermore, in some examples, imaging times are reduced compared to the other examples of the SiMREPS technology. For instance, the same number of binding and dissociation events occur in 1 to 10 seconds in some examples of intramolecular SiMREPS experiments as occur in 10 minutes in other examples of the SiMREPS technology. The intramolecular SiMREPS technology provides for the parallelization of experiments through spatial segregation. In some examples, the intramolecular SiMREPS technology reduces the concentrations of query probe that provide efficient detection. The intramolecular SiMREPS technology provides a platform that, in some examples, comprises many different Capture/Query probes immobilized within different regions of the imaging surface in a manner specified by the Address strand. As an example, one might use a standard microarray chip containing thousands of distinct sequences; these sequences could serve as the Address strands for immobilization of SiMREPS Capture/Query probes, permitting SiMREPS assays of thousands of target sequences (microRNAs, lncRNAs, DNA converted to single-stranded form) on a single chip.

Notably, in some examples, the Address strands are not related in sequence to any of the targets, since interaction occurs indirectly through the query and capture probes. Indeed, the Address strands are not required to be related to the targets. Furthermore, examples provide that the query and capture probes comprise affinity reagents other than DNA sequences, such as the dCas9/gRNA complexes discussed elsewhere in this application.

Examples provide control of exposure of the fluorophores to excitation sources, e.g., to reduce or minimize photobleaching prior to analysis. In some examples, kinetic signatures provide a correction mechanism to identify and correct false positive detections resulting from, e.g., deposit of a Capture/Query probe on the wrong part of the imaging surface (outside of its Address region). Examples also provide a technology in which false positives are minimized or reduced by splitting the Query and Capture probes into two non-contiguous probes that co-localize upon binding to the Address sequence (see, e.g., Fig. 5 b).

**Fluorescent moieties**

In some examples, a nucleic acid comprises a fluorescent moiety (e.g., a fluorogenic dye, also referred to as a "fluorophore" or a "fluor"). A wide variety of fluorescent moieties is known in the art and methods are known for linking a fluorescent moiety to a nucleotide prior to incorporation of the nucleotide into an oligonucleotide and for adding a fluorescent moiety to an oligonucleotide after synthesis of the oligonucleotide.

Examples of compounds that may be used as the fluorescent moiety include but are not limited to xanthene, anthracene, cyanine, porphyrin, and coumarin dyes. Examples of xanthene dyes that find use with the present technology include but are not limited to fluorescein, 6-carboxyfluorescein (6-FAM), 5-carboxyfluorescein (5-FAM), 5- or 6-carboxy-4, 7, 2', 7'- tetrachlorofluorescein (TET), 5- or 6-carboxy-4'5'2'4'5'7' hexachlorofluorescein (HEX), 5' or 6'-carboxy-4',5'-dichloro-2,'7'-dimethoxyfluorescein (JOE), 5-carboxy-2',4',5',7'-tetrachlorofluorescein (ZOE), rhodol, rhodamine, tetramethylrhodamine (TAMRA), 4,7-dlchlorotetramethyl rhodamine (DTAMRA), rhodamine X (ROX), and Texas Red. Examples of cyanine dyes that may find use with the present invention include but are not limited to Cy 3, Cy 3B, Cy 3.5, Cy 5, Cy 5.5, Cy 7, and Cy 7.5. Other fluorescent moieties and/or dyes that find use with the present technology include but are not limited to energy transfer dyes, composite dyes, and other aromatic compounds that give fluorescent signals. In some examples, the fluorescent moiety comprises a quantum dot.

In some examples, the fluorescent moiety comprises a fluorescent protein (e.g., a green fluorescent protein (GFP), a modified derivative of GFP (e.g., a GFP comprising S65T, an enhanced GFP (e.g., comprising F64L)), or others known in the art such as, e.g., blue fluorescent protein (e.g., EBFP, EBFP2, Azurite, mKalama1), cyan fluorescent protein (e.g., ECFP, Cerulean, CyPet, mTurquoise2), and yellow fluorescent protein derivatives (e.g., YFP, Citrine, Venus, YPet). Examples provide that the fluorescent protein may be covalently or noncovalently bonded to one or more query and/or capture probes.

Fluorescent dyes include, without limitation, d-Rhodamine acceptor dyes including Cy 5, dichloro[R110], dichloro[R6G], dichloro[TAMRA], dichloro[ROX] or the like, fluorescein donor dyes including fluorescein, 6-FAM, 5-FAM, or the like; Acridine including Acridine orange, Acridine yellow, Proflavin, pH 7, or the like; Aromatic Hydrocarbons including 2-Methylbenzoxazole, Ethyl p-dimethylaminobenzoate, Phenol, Pyrrole, benzene, toluene, or the like; Arylmethine Dyes including Auramine O, Crystal violet, Crystal violet, glycerol, Malachite Green or the like; Coumarin dyes including 7-Methoxycoumarin-4-acetic acid, Coumarin 1, Coumarin 30, Coumarin 314, Coumarin 343, Coumarin 6 or the like; Cyanine Dyes including 1,1'-diethyl-2,2'-cyanine iodide, Cryptocyanine, Indocarbocyanine (C3) dye, Indodicarbocyanine (C5) dye, Indotricarbocyanine (C7) dye, Oxacarbocyanine (C3) dye, Oxadicarbocyanine (C5) dye, Oxatricarbocyanine (C7) dye, Pinacyanol iodide, Stains all, Thiacarbocyanine (C3) dye, ethanol, Thiacarbocyanine (C3) dye, n-propanol, Thiadicarbocyanine (C5) dye, Thiatricarbocyanine (C7) dye, or the like; Dipyrrin dyes including N,N'-Difluoroboryl-1,9-dimethyl-5-(4-iodophenyl)-dipyrrin, N,N'-Difluoroboryl-1,9-dimethyl-5-[(4-(2-trimethylsilylethynyl), N,N'-Difluoroboryl-1,9-dimethyl-5-phenydipyrrin, or the like; Merocyanines including 4-(dicyanomethylene)-2-methyl-6-(p-dimethylaminostyryl)-4H-pyran (DCM), acetonitrile, 4-(dicyanomethylene)-2-methyl-6-(p-dimethylaminostyryl)-4H-pyran (DCM), methanol, 4-Dimethylamino-4'-nitrostilbene, Merocyanine 540, or the like; Miscellaneous Dyes including 4',6-Diamidino-2-phenylindole (DAPI), dimethylsulfoxide, 7-Benzylamino-4-nitrobenz-2-oxa-1,3-diazole, Dansyl glycine, Dansyl glycine, dioxane, Hoechst 33258, DMF, Hoechst 33258, Lucifer yellow CH, Piroxicam, Quinine sulfate, Quinine sulfate, Squarylium dye III, or the like; Oligophenylenes including 2,5-Diphenyloxazole (PPO), Biphenyl, POPOP, p-Quaterphenyl, p-Terphenyl, or the like; Oxazines including Cresyl violet perchlorate, Nile Blue, methanol, Nile Red, ethanol, Oxazine 1, Oxazine 170, or the like; Polycyclic Aromatic Hydrocarbons including 9,10-Bis(phenylethynyl)anthracene, 9,10-Diphenylanthracene, Anthracene, Naphthalene, Perylene, Pyrene, or the like; polyene/polyynes including 1,2-diphenylacetylene, 1,4-diphenylbutadiene, 1,4-diphenylbutadiyne, 1,6-Diphenylhexatriene, Beta-carotene, Stilbene, or the like; Redoxactive Chromophores including Anthraquinone, Azobenzene, Benzoquinone, Ferrocene, Riboflavin, Tris(2,2'-bipyridyl)ruthenium(II), Tetrapyrrole, Bilirubin, Chlorophyll a, diethyl ether, Chlorophyll a, methanol, Chlorophyll b, Diprotonatedtetraphenylporphyrin, Hematin, Magnesium octaethylporphyrin, Magnesium octaethylporphyrin (MgOEP), Magnesium phthalocyanine (MgPc), PrOH, Magnesium phthalocyanine (MgPc), pyridine, Magnesium tetramesitylporphyrin (MgTMP), Magnesium tetraphenylporphyrin (MgTPP), Octaethylporphyrin, Phthalocyanine (Pc), Porphin, ROX, TAMRA, Tetra-t-butylazaporphine, Tetra-t-butylnaphthalocyanine, Tetrakis(2,6-dichlorophenyl)porphyrin, Tetrakis(o-aminophenyl)porphyrin, Tetramesitylporphyrin (TMP), Tetraphenylporphyrin (TPP), Vitamin B12, Zinc octaethylporphyrin (ZnOEP), Zinc phthalocyanine (ZnPc), pyridine, Zinc tetramesitylporphyrin (ZnTMP), Zinc tetramesitylporphyrin radical cation, Zinc tetraphenylporphyrin (ZnTPP), or the like; Xanthenes including Eosin Y, Fluorescein, basic ethanol, Fluorescein, ethanol, Rhodamine 123, Rhodamine 6G, Rhodamine B, Rose bengal, Sulforhodamine 101, or the like; or mixtures or combination thereof or synthetic derivatives thereof.

Several classes of fluorogenic dyes and specific compounds are known that are appropriate for particular examples of the technology: xanthene derivatives such as fluorescein, rhodamine, Oregon green, eosin, and Texas red; cyanine derivatives such as cyanine, indocarbocyanine, oxacarbocyanine, thiacarbocyanine, and merocyanine; naphthalene derivatives (dansyl and prodan derivatives); coumarin derivatives; oxadiazole derivatives such as pyridyloxazole, nitrobenzoxadiazole, and benzoxadiazole; pyrene derivatives such as cascade blue; oxazine derivatives such as Nile red, Nile blue, cresyl violet, and oxazine 170; acridine derivatives such as proflavin, acridine orange, and acridine yellow; arylmethine derivatives such as auramine, crystal violet, and malachite green; and tetrapyrrole derivatives such as porphin, phtalocyanine, bilirubin. In some examples the fluorescent moiety a dye that is xanthene, fluorescein, rhodamine, BODIPY, cyanine, coumarin, pyrene, phthalocyanine, phycobiliprotein, ALEXA FLUOR® 350, ALEXA FLUOR® 405, ALEXA FLUOR® 430, ALEXA FLUOR® 488, ALEXA FLUOR® 514, ALEXA FLUOR® 532, ALEXA FLUOR® 546, ALEXA FLUOR® 555, ALEXA FLUOR® 568, ALEXA FLUOR® 568, ALEXA FLUOR® 594, ALEXA FLUOR® 610, ALEXA FLUOR® 633, ALEXA FLUOR® 647, ALEXA FLUOR® 660, ALEXA FLUOR® 680, ALEXA FLUOR® 700, ALEXA FLUOR® 750, or a squaraine dye. In some examples, the label is a fluorescently detectable moiety as described in, e.g., Haugland (September 2005) MOLECULAR PROBES HANDBOOK OF FLUORESCENT PROBES AND RESEARCH CHEMICALS (10th ed.).

In some examples the label (e.g., a fluorescently detectable label) is one available from ATTO-TEC GmbH (Am Eichenhang 50, 57076 Siegen, Germany), e.g., as described in U.S. Pat. Appl. Pub. Nos. 20110223677, 20110190486, 20110172420, 20060179585, and 20030003486; and in U.S. Pat. No. 7,935,822 (e.g., ATTO 390, ATTO 425, ATTO 465, ATTO 488, ATTO 495, ATTO 514, ATTO 520, ATTO 532, ATTO Rho6G, ATTO 542, ATTO 550, ATTO 565, ATTO Rho3B, ATTO Rho11, ATTO Rho12, ATTO Thio12, ATTO Rho101, ATTO 590, ATTO 594, ATTO Rho13, ATTO 610, ATTO 620, ATTO Rho14, ATTO 633, ATTO 647, ATTO 647N, ATTO 655, ATTO Oxa12, ATTO 665, ATTO 680, ATTO 700, ATTO 725, ATTO740).

One of ordinary skill in the art will recognize that dyes having emission maxima outside these ranges may be used as well. In some cases, dyes ranging between 500 nm to 700 nm have the advantage of being in the visible spectrum and can be detected using existing photomultiplier tubes. In some examples, the broad range of available dyes allows selection of dye sets that have emission wavelengths that are spread across the detection range. Detection systems capable of distinguishing many dyes are known in the art.

**Samples**

In some examples, nucleic acids (e.g., DNA or RNA) are isolated from a biological sample containing a variety of other components, such as proteins, lipids, and non-template nucleic acids. Nucleic acid template molecules can be obtained from any material (e.g., cellular material (live or dead), extracellular material, viral material, environmental samples (e.g., metagenomic samples), synthetic material (e.g., amplicons such as provided by PCR or other amplification technologies)), obtained from an animal, plant, bacterium, archaeon, fungus, or any other organism. Biological samples for use in the present technology include viral particles or preparations thereof. Nucleic acid molecules can be obtained directly from an organism or from a biological sample obtained from an organism, e.g., from blood, urine, cerebrospinal fluid, seminal fluid, saliva, sputum, stool, hair, sweat, tears, skin, and tissue. Exemplary samples include, but are not limited to, whole blood, lymphatic fluid, serum, plasma, buccal cells, sweat, tears, saliva, sputum, hair, skin, biopsy, cerebrospinal fluid (CSF), amniotic fluid, seminal fluid, vaginal excretions, serous fluid, synovial fluid, pericardial fluid, peritoneal fluid, pleural fluid, transudates, exudates, cystic fluid, bile, urine, gastric fluids, intestinal fluids, fecal samples, and swabs, aspirates (e.g., bone marrow, fine needle, etc.), washes (e.g., oral, nasopharyngeal, bronchial, bronchialalveolar, optic, rectal, intestinal, vaginal, epidermal, etc.), and/or other specimens.

Any tissue or body fluid specimen may be used as a source for nucleic acid for use in the technology, including forensic specimens, archived specimens, preserved specimens, and/or specimens stored for long periods of time, e.g., fresh-frozen, methanol/acetic acid fixed, or formalin-fixed paraffin embedded (FFPE) specimens and samples. Nucleic acid template molecules can also be isolated from cultured cells, such as a primary cell culture or a cell line. The cells or tissues from which template nucleic acids are obtained can be infected with a virus or other intracellular pathogen. A sample can also be total RNA extracted from a biological specimen, a cDNA library, viral, or genomic DNA. A sample may also be isolated DNA from a non-cellular origin, e.g. amplified/isolated DNA that has been stored in a freezer.

Nucleic acid molecules can be obtained, e.g., by extraction from a biological sample, e.g., by a variety of techniques such as those described by Maniatis, et al. (1982) Molecular Cloning: A Laboratory Manual, Cold Spring Harbor, N.Y. (see, e.g., pp. 280-281).

In some examples, the technology provides for the size selection of nucleic acids, e.g., to remove very short fragments or very long fragments.

In some examples, the technology is used to identify a nucleic acid in situ. In particular, examples of the technology provide for the identification of a nucleic acid directly in a tissue, cell, etc. (e.g., after permeabilizing the tissue, cell, etc.) without extracting the nucleic acid from the tissue, cell, etc. In some examples of the technology related to in situ detection, the technology is applied in vivo, ex vivo, and/or in vitro. In some examples, the sample is a crude sample, a minimally treated cell lysates, or a biofluid lysate. In some xamples, the nucleic acid is detected in a crude lysates without nucleic acid purification.

**Kits**

Some examples are related to kits for the detection of a nucleic acid. For instance, in some examples are provided a kit comprising a solid support (e.g., a microscope slide, a bead, a coverslip, an avidin (e.g., streptavidin)-conjugated microscope slide or coverslip, a solid support comprising a zero mode waveguide array, or the like), a dCas9/gRNA (e.g., comprising a biotinylated dCas9), and a query probe as described herein. Some examples further provide a non-biotinylated dCas9/gRNA.

Some examples further provide software on a computer-readable format or downloadable from the internet for the collection and analysis of query probe binding events and dwell times as described herein. In some examples, kits for multiplex detection comprise two or more query probes each comprising a sequence complementary to distinct query regions of one or more target nucleic acids and each comprising a different fluorescent moiety. In some examples, query probes are complementary to query regions of one or more nucleic acid targets. Some examples of kits comprise one or more positive controls and/or one or more negative controls. Some examples comprise a series of controls having known concentrations, e.g., to produce a standard curve of concentrations.

**Systems**

Some examples of the technology provide systems for the detection and quantification of a target nucleic acid. Systems according to the technology comprise, e.g., a solid support (e.g., a microscope slide, a coverslip, an avidin (e.g., streptavidin)-conjugated microscope slide or coverslip, a solid support comprising a zero mode waveguide array, or the like), a dCas9/gRNA (e.g., comprising a biotinylated dCas9), and a query probe as described herein. Some examples further provide a non-biotinylated dCas9/gRNA.

Some examples further comprise a fluorescence microscope comprising an illumination configuration to excite bound query probes (e.g., a prism-type total internal reflection fluorescence (TIRF) microscope, an objective-type TIRF microscope, a near-TIRF or HiLo microscope, a confocal laser scanning microscope, a zero-mode waveguide, and/or an illumination configuration capable of parallel monitoring of a large area of the slide or coverslip (> 100 $\mu m^2$) while restricting illumination to a small region of space near the surface). Some examples comprise a fluorescence detector, e.g., a detector comprising an intensified charge coupled device (ICCD), an electron-multiplying charge coupled device (EM-CCD), a complementary metal-oxide-semiconductor (CMOS), a photomultiplier tube (PMT), an avalanche photodiode (APD), and/or another detector capable of detecting fluorescence emission from single chromophores. Some examples comprise a computer and software encoding instructions for the computer to perform.

Some examples comprise optics, such as lenses, mirrors, dichroic mirrors, optical filters, etc., e.g., to detect fluorescence selectively within a specific range of wavelengths or multiple ranges of wavelengths.

For example, computer-based analysis software may be used to translate the raw data generated by the detection assay (e.g., the presence, absence, or amount of one or more nucleic acids (e.g., one or more biomarkers) into data of predictive value for a clinician. The clinician can access the predictive data using any suitable means.

For instance, a computer system may be comprised upon which examples of the present technology may be implemented. In various examples, a computer system includes a bus or other communication mechanism for communicating information and a processor coupled with the bus for processing information. In various examples, the computer system includes a memory, which can be a random access memory (RAM) or other dynamic storage device, coupled to the bus, and instructions to be executed by the processor. Memory also can be used for storing temporary variables or other intermediate information during execution of instructions to be executed by the processor. In various examples, the computer system can further include a read only memory (ROM) or other static storage device coupled to the bus for storing static information and instructions for the processor. A storage device, such as a magnetic disk or optical disk, can be provided and coupled to the bus for storing information and instructions.

In various examples, the computer system is coupled via the bus to a display, such as a cathode ray tube (CRT) or a liquid crystal display (LCD), for displaying information to a computer user. An input device, including alphanumeric and other keys, can be coupled to the bus for communicating information and command selections to the processor. Another type of user input device is a cursor control, such as a mouse, a trackball, or cursor direction keys for communicating direction information and command selections to the processor and for controlling cursor movement on the display. This input device typically has two degrees of freedom in two axes, a first axis (e.g., x) and a second axis (e.g., y), that allows the device to specify positions in a plane.

A computer system can perform aspects of the present technology. Consistent with certain implementations of the present technology, results can be provided by the computer system in response to the processor executing one or more sequences of one or more instructions contained in the memory. Such instructions can be read into the memory from another computer-readable medium, such as a storage device. Execution of the sequences of instructions contained in the memory can cause the processor to perform the methods described herein. Alternatively, hard-wired circuitry can be used in place of or in combination with software instructions to implement the present teachings. Thus, implementations of the present technology are not limited to any specific combination of hardware circuitry and software.

The term "computer-readable medium" as used herein refers to any media that participates in providing instructions to the processor for execution. Such a medium can take many forms, including but not limited to, non-volatile media, volatile media, and transmission media. Examples of non-volatile media can include, but are not limited to, optical or magnetic disks, such as a storage device. Examples of volatile media can include, but are not limited to, dynamic memory. Examples of transmission media can include, but are not limited to, coaxial cables, copper wire, and fiber optics, including the wires that comprise the bus.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, PROM, and EPROM, a FLASH-EPROM, any other memory chip or cartridge, or any other tangible medium from which a computer can read.

Various forms of computer readable media can be involved in carrying one or more sequences of one or more instructions to the processor for execution. For example, the instructions can initially be carried on the magnetic disk of a remote computer. The remote computer can load the instructions into its dynamic memory and send the instructions over a network connection (e.g., a LAN, a WAN, the internet, a telephone line). A local computer system can receive the data and transmit it to the bus. The bus can carry the data to the memory, from which the processor retrieves and executes the instructions. The instructions received by the memory may optionally be stored on a storage device either before or after execution by the processor.

In accordance with various examples, instructions configured to be executed by a processor to perform a method are stored on a computer-readable medium. The computer-readable medium can be a device that stores digital information. For example, a computer-readable medium includes a compact disc read-only memory (CD-ROM) as is known in the art for storing software. The computer-readable medium is accessed by a processor suitable for executing instructions configured to be executed.

In accordance with such a computer system, some examples of the technology provided herein further comprise functionalities for collecting, storing, and/or analyzing data (e.g., presence, absence, concentration of a nucleic acid). For example, some examples contemplate a system that comprises a processor, a memory, and/or a database for, e.g., storing and executing instructions, analyzing fluorescence, image data, performing calculations using the data, transforming the data, and storing the data. In some examples, an algorithm applies a statistical model (e.g., a Poisson model or hidden Markov model) to the data.

Many diagnostics involve determining the presence of, or a nucleotide sequence of, one or more nucleic acids (e.g., a nucleic acid biomarker). Thus, in some examples, an equation comprising variables representing the presence, absence, concentration, amount, or sequence properties of multiple nucleic acids produces a value that finds use in making a diagnosis or assessing the presence or qualities of a nucleic acid. As such, in some examples this value is presented by a device, e.g., by an indicator related to the result (e.g., an LED, an icon on a display, a sound, or the like). In some examples, a device stores the value, transmits the value, or uses the value for additional calculations. In some examples, an equation comprises variables representing the presence, absence, concentration, amount, or sequence properties of one or more of a methylated locus in genomic DNA, a microRNA, a mutant gene biomarker, or a chromosomal aberration.

Thus, in some examples, the present technology provides the further benefit that a clinician, who is not likely to be trained in genetics or molecular biology, need not understand the raw data. The data are presented directly to the clinician in its most useful form. The clinician is then able to utilize the information to optimize the care of a subject. Any method capable of receiving, processing, and transmitting the information to and from laboratories conducting the assays, information providers, medical personal, and/or subjects is contemplated. For example, in some examples of the present technology, a sample is obtained from a subject and submitted to a profiling service (e.g., a clinical lab at a medical facility, genomic profiling business, etc.), located in any part of the world (e.g., in a country different than the country where the subject resides or where the information is ultimately used) to generate raw data. Where the sample comprises a tissue or other biological sample, the subject may visit a medical center to have the sample obtained and sent to the profiling center or subjects may collect the sample themselves and directly send it to a profiling center. Where the sample comprises previously determined biological information, the information may be directly sent to the profiling service by the subject (e.g., an information card containing the information may be scanned by a computer and the data transmitted to a computer of the profiling center using electronic communication systems). Once received by the profiling service, the sample is processed and a profile is produced that is specific for the diagnostic or prognostic information desired for the subject. The profile data are then prepared in a format suitable for interpretation by a treating clinician. For example, rather than providing raw expression data, the prepared format may represent a diagnosis or risk assessment for the subject, along with recommendations for particular treatment options. The data may be displayed to the clinician by any suitable method. For example, in some examples, the profiling service generates a report that can be printed for the clinician (e.g., at the point of care) or displayed to the clinician on a computer monitor. In some examples, the information is first analyzed at the point of care or at a regional facility. The raw data are then sent to a central processing facility for further analysis and/or to convert the raw data to information useful for a clinician or patient. The central processing facility provides the advantage of privacy (all data are stored in a central facility with uniform security protocols), speed, and uniformity of data analysis. The central processing facility can then control the fate of the data following treatment of the subject. For example, using an electronic communication system, the central facility can provide data to the clinician, the subject, or researchers. In some examples, the subject is able to access the data using the electronic communication system. The subject may chose further intervention or counseling based on the results. In some examples, the data are used for research use. For example, the data may be used to further optimize the inclusion or elimination of markers as useful indicators of a particular condition associated with the disease.

**Claims**

1. A complex for detecting a target nucleic acid, the complex comprising:

   1) a target nucleic acid comprising a first region adjacent to a second region;
   2) a detectably labeled capture probe hybridized to the first region;
   3) a query probe labeled with a quencher or fluorescent acceptor compatible with the label of the capture probe, wherein the query probe hybridizes repeatedly to the second region with a kinetic rate constant $k_{off}$ that is greater than 0.1 $min^{-1}$ and/or a kinetic rate constant $k_{on}$ that is greater than 0.1 $min^{-1}$.

2. The complex of claim 1, wherein the capture probe and the query probe are linked to provide an intramolecular probe.

3. The complex of claim 1, wherein the target nucleic acid binds with thermodynamic stability to the capture sequence and the target nucleic acid undergoes transient binding with the query probe.

4. The complex of claim 1, further comprising an address strand that binds the query probe and the capture probe to a surface.

5. The complex of claim 4, wherein a microarray comprises the address strand.

6. The complex of claim 4, wherein the address strand is not related in sequence to the target nucleic acid, since interaction occurs indirectly through the query and capture probe.

7. A method for detecting a target nucleic acid, the method comprising:

   a) hybridizing a detectably labeled capture probe to a first region of a target nucleic acid comprising said first region adjacent to a second region;
   b) providing a query probe labeled with a quencher or fluorescent acceptor compatible with the label of the capture probe,
   wherein the query probe hybridizes repeatedly to the second region with a kinetic rate constant $k_{off}$ that is greater than 0.1 $min^{-1}$ and/or a kinetic rate constant $k_{on}$ that is greater than 0.1 $min^{-1}$; and
   c) recording a time-dependent change in fluorescence intensity of the query probe label.

8. The method of claim 7, wherein the capture probe and the query probe are linked to provide an intramolecular probe.

9. The method of claim 7, wherein:

   - a number of transitions in the time-dependent change in fluorescence intensity is used to discriminate the target nucleic from background signal, non-target nucleic acid, and/or spurious binding of the query probe;
   - a dwell time is used to identify the presence of the target nucleic acid;
   - a statistical analysis of the time-dependent change in fluorescence intensity is used to detect, quantify, and/or identify the target nucleic acid; and/or
   - a pattern of time-dependent changes in the fluorescence intensity is used to detect, quantify, and/or identify the target nucleic acid.

10. The method of claim 7, further comprising an address strand that binds the query probe and the capture probe to a surface.

11. The method of claim 10, wherein a microarray comprises the address strand.

12. The method of claim 7, wherein recording a time-dependent change in fluorescence intensity of the query probe label comprises imaging.

13. The method of claim 7, wherein the time-dependent change in fluorescence intensity of the query probe label is recorded for 1 to 10 seconds.

14. The method of claim 10, wherein said microarray comprises a plurality of different capture and query probes immobilized within different regions of the imaging surface.

**15.** The method of claim 14, wherein said method provides for the parallelization of experiments through spatial segregation.

**Patentansprüche**

**1.** Komplex zum Detektieren einer Zielnukleinsäure, wobei der Komplex umfasst:

1) eine Zielnukleinsäure, die eine erste Region angrenzend an eine zweite Region umfasst;
2) eine nachweisbar markierte Einfangsonde, die an die erste Region hybridisiert ist;
3) eine Abfragesonde, die mit einem Quencher oder Fluoreszenzakzeptor markiert ist, der mit der Markierung der Einfangsonde kompatibel ist, wobei die Abfragesonde wiederholt mit der zweiten Region mit einer kinetischen Geschwindigkeitskonstante $k_{off}$, die größer als 0,1 $min^{-1}$ ist, und/oder einer kinetischen Geschwindigkeitskonstante $k_{on}$, die größer als 0,1 $min^{-1}$ ist, hybridisiert.

**2.** Komplex nach Anspruch 1, wobei die Einfangsonde und die Abfragesonde miteinander verbunden sind, um eine intramolekulare Sonde bereitzustellen.

**3.** Komplex nach Anspruch 1, wobei die Zielnukleinsäure mit thermodynamischer Stabilität an die Einfangsequenz bindet und die Zielnukleinsäure eine transiente Bindung mit der Abfragesonde eingeht.

**4.** Komplex nach Anspruch 1, des Weiteren umfassend einen Adressierstrang, der die Abfragesonde und die Einfangsonde an eine Oberfläche bindet.

**5.** Komplex nach Anspruch 4, wobei ein Microarray den Adressierstrang umfasst.

**6.** Komplex nach Anspruch 4, wobei der Adressierstrang in seiner Sequenz nicht mit der Zielnukleinsäure verwandt ist, da die Wechselwirkung indirekt über die Abfrage- und Einfangsonde erfolgt.

**7.** Verfahren zum Nachweis einer Zielnukleinsäure, wobei das Verfahren umfasst:

a) Hybridisierung einer nachweisbar markierten Einfangsonde an eine erste Region einer Zielnukleinsäure, die eine erste Region angrenzend an eine zweite Region umfasst;
b) Bereitstellen einer Abfragesonde, die mit einem Quencher oder Fluoreszenzakzeptor markiert ist, der mit der Markierung der Einfangsonde kompatibel ist,
wobei die Abfragesonde wiederholt mit der zweiten Region mit einer kinetischen Geschwindigkeitskonstante $k_{off}$, die größer als 0,1 $min^{-1}$ ist, und/oder einer kinetischen Geschwindigkeitskonstante $k_{on}$, die größer als 0,1 $min^{-1}$ ist, hybridisiert; und
c) Aufzeichnen einer zeitabhängigen Änderung der Fluoreszenzintensität der Abfragesondenmarkierung.

**8.** Verfahren nach Anspruch 7, wobei die Einfangsonde und die Abfragesonde miteinander verbunden sind, um eine intramolekulare Sonde bereitzustellen.

**9.** Verfahren nach Anspruch 7, wobei:

- eine Anzahl von Übergängen in der zeitabhängigen Änderung der Fluoreszenzintensität verwendet wird, um die Zielnukleinsäure von einem Hintergrundsignal, einer Nicht-Zielnukleinsäure und/oder einer fehlerhaften Bindung der Abfragesonde zu unterscheiden;
- eine Verweilzeit verwendet wird, um das Vorhandensein der Zielnukleinsäure zu identifizieren;
- eine statistische Analyse der zeitabhängigen Änderung der Fluoreszenzintensität verwendet wird, um die Zielnukleinsäure nachzuweisen, zu quantifizieren und/oder zu identifizieren; und/oder
- ein Muster von zeitabhängigen Änderungen der Fluoreszenzintensität zum Nachweis, zur Quantifizierung und/oder zur Identifizierung der Zielnukleinsäure verwendet wird.

**10.** Verfahren nach Anspruch 7, des Weiteren umfassend einen Adressierstrang, der die Abfragesonde und die Einfangsonde an eine Oberfläche bindet.

**11.** Verfahren nach Anspruch 10, wobei ein Microarray den Adressierstrang umfasst.

**12.** Verfahren nach Anspruch 7, wobei die Aufzeichnung einer zeitabhängigen Änderung der Fluoreszenzintensität der Abfragesondenmarkierung die Bildgebung umfasst.

**13.** Verfahren nach Anspruch 7, wobei die zeitabhängige Änderung der Fluoreszenzintensität der Abfragesondenmarkierung 1 bis 10 Sekunden lang aufgezeichnet wird.

**14.** Verfahren nach Anspruch 10, wobei der Microarray eine Vielzahl von verschiedenen Einfang- und Abfragesonden umfasst, die in verschiedenen Bereichen der Abbildungsoberfläche immobilisiert sind.

**15.** Verfahren nach Anspruch 14, wobei das Verfahren für die Parallelisierung von Experimenten durch räumliche Trennung sorgt.

**Revendications**

**1.** Complexe pour détecter un acide nucléique cible, le complexe comprenant :

1) un acide nucléique cible comprenant une première région adjacente à une deuxième région ;
2) une sonde de capture marquée de façon détectable hybridée à la première région ;
3) une sonde d'interrogation marquée avec un désactiveur ou un accepteur fluorescent compatible avec le marqueur de la sonde de capture,

dans lequel la sonde d'interrogation s'hybride de façon répétée à la deuxième région avec une constante cinétique koff qui est supérieure à 0,1 min-1 et/ou une constante cinétique kon qui est supérieure à 0,1 min-1.

**2.** Complexe selon la revendication 1, dans lequel la sonde de capture et la sonde d'interrogation sont liées pour fournir une sonde intramoléculaire.

**3.** Complexe selon la revendication 1, dans lequel l'acide nucléique cible se lie avec une stabilité thermodynamique à la séquence de capture et l'acide nucléique cible subit une liaison transitoire à la sonde d'interrogation.

**4.** Complexe selon la revendication 1, comprenant en outre un brin d'adresse qui lie la sonde d'interrogation et la sonde de capture à une surface.

**5.** Complexe selon la revendication 4, dans lequel un micro-réseau comprend le brin d'adresse.

**6.** Complexe selon la revendication 4, dans lequel le brin d'adresse n'est pas apparenté en termes de séquence à l'acide nucléique cible, étant donné qu'une interaction se produit indirectement par l'intermédiaire des sondes d'interrogation et de capture.

**7.** Procédé de détection d'un acide nucléique cible, le procédé comprenant :

a) l'hybridation d'une sonde de capture marquée de façon détectable à une première région d'un acide nucléique cible comprenant ladite première région adjacente à une deuxième région
b) la fourniture d'une sonde d'interrogation marquée avec un désactiveur ou un accepteur fluorescent compatible avec le marqueur de la sonde de capture,
dans lequel la sonde d'interrogation s'hybride de façon répétée à la deuxième région avec une constante cinétique koff qui est supérieure à 0,1 min-1 et/ou une constante cinétique kon qui est supérieure à 0,1 min-1 ; et
c) l'enregistrement d'un changement d'intensité de fluorescence en fonction du temps du marqueur de sonde d'interrogation.

**8.** Procédé selon la revendication 7, dans lequel la sonde de capture et la sonde d'interrogation sont liées pour fournir une sonde intramoléculaire.

**9.** Procédé selon la revendication 7, dans lequel :

- un nombre de transitions dans le changement d'intensité de fluorescence en fonction du temps est utilisé pour distinguer l'acide nucléique cible du signal d'arrière-plan, d'un acide nucléique non-cible et/ou d'une liaison parasite de la sonde d'interrogation ;

- un temps de maintien est utilisé pour identifier la présence de l'acide nucléique cible ;

- une analyse statistique du changement d'intensité de fluorescence en fonction du temps est utilisée pour détecter, quantifier et/ou identifier l'acide nucléique cible ; et/ou

- un profil de changements en fonction du temps de l'intensité de fluorescence est utilisé pour détecter, quantifier et/ou identifier l'acide nucléique cible.

10. Procédé selon la revendication 7, comprenant en outre un brin d'adresse qui lie la sonde d'interrogation et la sonde de capture à une surface.

11. Procédé selon la revendication 10, dans lequel un micro-réseau comprend le brin d'adresse.

12. Procédé selon la revendication 7, dans lequel l'enregistrement d'un changement d'intensité de fluorescence en fonction du temps du marqueur de sonde d'interrogation comprend une imagerie.

13. Procédé selon la revendication 7, dans lequel le changement d'intensité de fluorescence en fonction du temps du marqueur de sonde d'interrogation est enregistré pendant 1 à 10 secondes.

14. Procédé selon la revendication 10, dans lequel ledit microréseau comprend une pluralité de sondes de capture et d'interrogation différentes immobilisées dans différentes régions de la surface d'imagerie.

15. Procédé selon la revendication 14, ledit procédé permettant la parallélisation d'essais par ségrégation spatiale.

Figure 1

a

miRNA
target

hybridize at RT ↓

Fluorescent Probe
(9–10 nt)

biotinyl
LNA
capture
probe
(9–11 nt)

streptavidin
PEG

EP 3 800 249 B1

FIGURE 1 (cont.)

b

c

FIGURE 1 (cont.)

FIGURE 1 (cont.)

FIGURE 2

**FIGURE 2 (cont.)**

FIGURE 2 (cont.)

FIGURE 2 (cont.)

FIGURE 2 (cont.)

FIGURE 2 (cont.)

FIGURE 3

FIGURE 4

FIGURE 5

a

Query probe (transient)

Quencher
(or acceptor
fluorophore)

Donor
fluorophore

Capture probe
(stable)

Address Strand

Surface

target
nucleic
acid

Donor fluorescent

Donor quenched
(Acceptor fluorescent)

FIGURE 5 (cont.)

b

Query probe (transient)

Capture probe
(stable)

Surface

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 62293589
- US 20160046988 A1
- US 6001983 A, S. Benner
- US 20160010076 A
- US 20090084980 A
- EP 2300983 B1
- WO 2014018584 A1
- US 20110223677
- US 20110190486
- US 20110172420
- US 20060179585
- US 20030003486
- US 7935822 B
- EP 17750678
- US 2017016977 W
- US 62293589 B

## Non-patent literature cited in the description

- **JOHNSON-BUCK et al.** Kinetic fingerprinting to identify and count single nucleic acids. *Nat Biotechnol,* 2015, vol. 33, 730-2
- **KLEINSTIVER et al.** High-fidelity CRISPR-Cas9 nucleases with no detectable genome-wide off-target effects. *Nature,* 2016, vol. 529, 490-495
- **SLAYMAKER et al.** Rationally engineered Cas9 nucleases with improved specificity. *Science,* 2015, vol. 351, 84-8
- **ALBERT L. LEHNINGER.** Principles of Biochemistry. Worth Pub, 1982, 793-800
- **B. A. SCHWEITZER ; E. T. KOOL.** *J. Org. Chem.,* 1994, vol. 59, 7238-7242
- **B. A. SCHWEITZER ; E. T. KOOL.** *J. Am. Chem. Soc.,* 1995, vol. 117, 1863-1872
- **P. KONG et al.** *Nucleic Acids Res.,* 1989, vol. 17, 10373-10383
- **P. KONG et al.** *Nucleic Acids Res.,* 1992, vol. 20, 5149-5152
- **MARMUR ; LANE.** *Proc. Natl. Acad. Sci. USA,* 1960, vol. 46, 453
- **DOTY et al.** *Proc. Natl. Acad. Sci. USA,* 1960, vol. 46, 461
- **ANDERSON ; YOUNG.** Quantitative Filter Hybridization. *Nucleic Acid Hybridization,* 1985
- **ALLAWI ; SANTALUCIA.** *Biochemistry,* 1997, vol. 36, 10581-94
- *Nat Methods,* vol. 5, 475-89
- **JOHNSON-BUCKET.** Kinetic fingerprinting to identify and count single nucleic acids. *Nat Biotechnol,* 2015, vol. 33, 730-2
- **BARRANGOU et al.** *Science,* 2007, vol. 315, 1709-1712
- **DOUDNA et al.** The new frontier of genome engineering with CRISPR-Cas9. *Science,* 2014, vol. 346, 6213
- **JINEK et al.** A Programmable Dual-RNA-Guided DNA Endonuclease in Adaptive Bacterial Immunity. *Science,* 2012, vol. 337, 816-821
- **ZHANG et al.** Processing-independent CRISPR RNAs limit natural transformation in Neisseria meningitidis. *Molecular Cell,* 2013, vol. 50, 488-503
- **QI et al.** Repurposing CRISPR as an RNA-guided platform for sequence-specific control of gene expression. *Cell,* 2013, vol. 152 (5), 1173-83
- **RAN et al.** In vivo genome editing using Staphylococcus aureus Cas9. *Nature,* 2015, vol. 520, 186-191
- *RNA Biology,* 2013, vol. 10 (5), 1-12
- **ESVELT et al.** *Nat Methods,* November 2013, vol. 10 (11), 1116-21
- **FONFARA et al.** Phylogeny of Cas9 determines functional exchangeability of dual-RNA and Cas9 among orthologous type II CRISPR-Cas systems. *Nucleic Acids Res.,* 22 November 2013
- **CONG et al.** *Science,* 2013, vol. 339, 819
- **NISHIMASU.** *Cell,* 2014, vol. 156, 935-949
- **CHYLINSKI et al.** *RNA Biology,* 2013, vol. 10 (5), 1-12
- **FONFARA et al.** *Nucl. Acids Res.,* 2014, vol. 42 (4), 2577-2590
- **NEEDLEMAN ; WUNSCH.** *J. Mol. Biol.,* 1970, vol. 48, 444-453
- **DUDA et al.** Pattern classification. Wiley, 2001
- **BISHOP.** Pattern Recognition and Machine Learning. Springer, 2006
- *Pol J Pathol,* vol. 63 (3), 145-64

- **GERECKE et al.** Ultrasensitive detection of unknown colon cancer-initiating mutations using the example of the Adenomatous polyposis coli gene. *Cancer Prev Res (Phila),* 2013, vol. 6 (9), 898-907

- **O'CONNELL et al.** Programmable RNA recognition and cleavage by CRISPR/Cas9. *Nature,* 2014, vol. 516, 263-6